# EUROPEAN PATENT SPECIFICATION

(11) **EP 4 418 994 B1**
(45) Date of publication and mention of the grant of the patent: **25.11.2026**
(21) Application number: 22812911.0
(22) Date of filing: 24.10.2022
(51) Int. Cl.: A61B 5/06, A61B 5/00, A61B 34/20

(54) **REFERENCE PLANE FOR MEDICAL DEVICE PLACEMENT**
BEZUGSEBENE FÜR DIE PLATZIERUNG MEDIZINISCHER GERÄTE
PLAN DE RÉFÉRENCE POUR LE PLACEMENT DES DISPOSITIFS MÉDICAUX

(30) Priority: 25.10.2021 US 202163271630 P
(43) Date of publication of application: 28.08.2024
(73) Proprietor: Bard Access Systems, Inc., Salt Lake City, UT 84116 (US)
(72) Inventor: SOWARDS, Steffan, Salt Lake City, UT 84124 (US); MISENER, Anthony, K., Bountiful, UT 84010 (US); MCLAUGHLIN, William, Robert, Bountiful, UT 84010 (US)
(74) Representative: Hoffmann Eitle
(86) International application number: PCT/US2022/047538
(87) International publication number: WO 2023/076143

(56) References cited:
- US-A1- 2013 028 554
- US-A1- 2016 213 432
- US-A1- 2018 235 709
- US-A1- 2018 279 909
- US-A1- 2021 268 229

## Description

### PRIORITY

This application claims the benefit of priority to U.S. Provisional Application No. 63/271,630, filed October 25, 2021.

### BACKGROUND

In the past, certain intravascular guidance of medical devices, such as guidewires and catheters for example, have used fluoroscopic methods for tracking tips of the medical devices and determining whether distal tips are appropriately localized in their target anatomical structures. However, such fluoroscopic methods expose patients and their attending clinicians to harmful X-ray radiation. Moreover, in some cases, the patients are exposed to potentially harmful contrast media needed for the fluoroscopic methods. US 2018/0279909 A1 relates to a hub for an optical shape sensing reference including a hub body configured to receive an elongated flexible instrument with a shape sensing system coupled to the flexible instrument within a path formed in the hub body.
US 2018/0235709 A1 relates to a method of registering sets of anatomical data for use during a surgical procedure.
US 2013/0028554 A1 relates to systems and methods for integrating and/or registering a shape sensing fiber in or to various instruments.
US 2016/0213432 A1 relates to an optical shape sensing hub including a longitudinal body forming a cavity configured to receive two or more optical shape sensing (OSS) enabled instruments.

Disclosed herein is a fiber optic shape sensing system and methods performed thereby where the system is configured to display an image of three-dimensional shape of a medical device using optical fiber technology. Further, the system is configured to define a reference frame for the three-dimensional shape to enable to the clinician to view an image the three-dimensional shape according to defined orientations of the three-dimensional shape.

### SUMMARY OF THE INVENTION

The invention is defined in the appended set of claims.

SUMMARY OF THE DISCLOSURE Briefly summarized, disclosed herein is a medical device system generally including a medical device coupled with a console. The medical device includes an optical fiber having one or more of core fibers, each of the one or more core fibers including a plurality of sensors distributed along a longitudinal length of a corresponding core fiber, each sensor of the plurality of sensors configured to: (i) reflect a light signal of a different spectral width based on received incident light, and (ii) change a characteristic of the reflected light signal based on strain experienced by the optical fiber.

The console includes one or more processors and a non-transitory computer-readable medium having stored thereon logic, when executed by the one or more processors, causes performance of operations of the system. The operations include: (i) providing an incident light signal to the optical fiber, (ii) receiving reflected light signals of different spectral widths of the incident light by one or more of the plurality of sensors (iii) processing the reflected light signals associated with the one or more of core fibers to determine a three-dimensional (3D) shape of the optical fiber, (iv) detecting a predetermined subshape of the 3D shape, and (v) defining a reference plane in accordance with the predetermined subshape, where the reference plane defines a viewing perspective of the 3D shape. In further embodiments, the operations include rendering an image of the 3D shape on a display of the system in accordance with the reference plane.

In some embodiments, detecting the predetermined subshape includes comparing a subshape of the 3D shape with a stored predetermined subshape in memory and as a result of the comparison, identifying the subshape as the predetermined subshape of the 3D shape. A plurality of stored predetermined subshapes may be included in the memory, and the operations may further include selecting the stored predetermined subshape from the plurality of stored predetermined subshapes in memory.

The predetermined subshape may be defined by a predetermined pathway of the medical device. In some embodiments, the medical device is configured for insertion within a patient body, and the predetermined pathway includes a predetermined anatomical pathway of the medical device. The operations may include receiving input from a clinician defining the anatomical pathway for the medical device and selecting the stored predetermined subshape according to the input from the clinician. In some embodiments, the input from the clinician includes a location of an insertion site for the medical device. In some embodiments, the predetermined anatomical pathway may extend along one or more of a basilic vein, a subclavian vein, an innominate vein, or a superior vena cava of the patient.

In some embodiments, the predetermined subshape is defined by a predetermined pathway of the medical device external to the patient such as a predetermined pathway defined by a subshape guide of the medical device, where in use, the medical device is disposed within a pathway of the subshape guide. In some embodiments, the clinician may insert the medical device within the pathway of the subshape guide during use of the system.

The subshape guide may be included within a package of the medical device and in some embodiments, the subshape guide may be formed integral to the package. In use, the subshape guide may be attached to the patient to maintain an orientation of the predetermined subshape with respect to the patient.

In some embodiments, defining the reference plane includes identifying a pair of shape segments of the predetermined subshape and determining a plane in parallel with both shape segments.

In some embodiments, the system further includes a device guide including a lumen extending along a straight section of the device guide. In use, the medical device is inserted within the lumen, and the operations further include calibrating the optical fiber in accordance with the straight section.

In some embodiments, the medical device is one of an introducer wire, a guidewire, a stylet, a stylet within a needle, a needle with the optical fiber inlayed into a cannula of the needle or a catheter with the optical fiber inlayed into one or more walls of the catheter.

Also disclosed herein is a method for detecting placement of a medical device within a patient body. The method includes providing by a system an incident light signal to an optical fiber included within the medical device, wherein the optical fiber includes one or more of core fibers, each of the one or more of core fibers including a plurality of reflective gratings distributed along a longitudinal length of a corresponding core fiber and each of the plurality of reflective gratings being configured to (i) reflect a light signal of a different spectral width based on received incident light, and (ii) change a characteristic of the reflected light signal based on strain experienced by the optical fiber.

The method further includes: (i) receiving reflected light signals of different spectral widths of the incident light by one or more of the plurality of sensors, (ii) processing the reflected light signals associated with the one or more of core fibers to determine a three-dimensional (3D) shape of the optical fiber, (iii) detecting a predetermined subshape of the 3D shape, and (iv) defining a reference plane in accordance with the predetermined subshape, where the reference plane defines a viewing perspective of the 3D shape. In further embodiments, the method further includes rendering an image of the 3D shape on a display of the system in accordance with the reference plane.

In some embodiments, detecting the predetermined subshape includes comparing a subshape of the 3D shape with a stored predetermined subshape in memory of the system and as a result of the comparison, identifying the subshape as the predetermined subshape of the 3D shape. A plurality of stored predetermined subshapes may be included in memory of the system, and the method may further include selecting the stored predetermined subshape from the plurality of stored predetermined subshapes in memory.

In some embodiments of the method, the predetermined subshape is defined by a predetermined pathway of the medical device and the predetermined pathway may include a predetermined anatomical pathway of the medical device.

The method may further include receiving input from a clinician defining the anatomical pathway for the medical device and selecting the stored predetermined subshape according to the input from the clinician. The input from the clinician may include a location of an insertion site for the medical device. In some embodiments, the predetermined anatomical pathway extends along one or more of a basilic vein, a subclavian vein, an innominate vein, or a superior vena cava of the patient. In other embodiments of the method, the predetermined subshape is defined by a predetermined pathway of the medical device external to the patient.

The method may further include identifying a pair of shape segments of the predetermined subshape and determining a plane in parallel with both shape segments.

In some embodiments of the method, the medical device is inserted within a lumen of a medical device guide of the system, and the method further includes calibrating the optical fiber in accordance with a straight section of the medical device guide.

In some embodiments of the method, the medical device is one of an introducer wire, a guidewire, a stylet, a stylet within a needle, a needle with the optical fiber inlayed into a cannula of the needle or a catheter with the optical fiber inlayed into one or more walls of the catheter.

These and other features of the concepts provided herein will become more apparent to those of skill in the art in view of the accompanying drawings and following description, which disclose particular embodiments of such concepts in greater detail.

### BRIEF DESCRIPTION OF THE DRAWINGS

Embodiments of the disclosure are illustrated by way of example and not by way of limitation in the figures of the accompanying drawings, in which like references indicate similar elements and in which:
FIG. 1A is an illustrative embodiment of a medical instrument monitoring system including a medical instrument with optic shape sensing capabilities in accordance with some embodiments;
FIG. 1B is an alternative illustrative embodiment of the medical instrument monitoring system 100 in accordance with some embodiments;
FIG. 2 is an exemplary embodiment of a structure of a section of the multi-core optical fiber included within the stylet 120 of FIG. 1A in accordance with some embodiments;
FIG. 3A is a first exemplary embodiment of the stylet of FIG. 1A supporting both an optical and electrical signaling in accordance with some embodiments;
FIG. 3B is a cross sectional view of the stylet of FIG. 3A in accordance with some embodiments;
FIG. 4A is a second exemplary embodiment of the stylet of FIG. 1B in accordance with some embodiments;
FIG. 4B is a cross sectional view of the stylet of FIG. 4A in accordance with some embodiments;
FIG. 5A is an elevation view of a first illustrative embodiment of a catheter including integrated tubing, a diametrically disposed septum, and micro-lumens formed within the tubing and septum in accordance with some embodiments;
FIG. 5B is a perspective view of the first illustrative embodiment of the catheter of FIG. 5A including core fibers installed within the micro-lumens in accordance with some embodiments;
FIGS. 6A-6B are flowcharts of the methods of operations conducted by the medical instrument monitoring system of FIGS. 1A-1B to achieve optic 3D shape sensing, in accordance with some embodiments;
FIG. 7A is an exemplary embodiment of the medical instrument monitoring system of FIGS. 1A-1B during operation and insertion of the catheter into a patient in accordance with some embodiments;
FIG. 7B illustrates the 3D shape of FIG. 7A according to a reference plane in accordance with some embodiments;
FIG. 8 is an exemplary screen shot of image of the 3D shape of FIGS. 7A-7B in accordance with some embodiments;
FIG. 9A is an illustration of a second exemplary implementation of the system of FIGS. 1A-1B in accordance with some embodiments;
FIGS. 9B-9D illustrate various embodiments of the catheter of FIG. 9A including packaging of the catheter in accordance with some embodiments; and
FIG. 10 is a flowchart of a method of operations conducted by the medical instrument monitoring system of FIGS. 1A-1B to display an image of the 3D shape according to the reference plane in accordance with some embodiments.

### DETAILED DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a probe disclosed herein includes a portion of the probe intended to be near a clinician when the probe is used on a patient. Likewise, a "proximal length" of, for example, the probe includes a length of the probe intended to be near the clinician when the probe is used on the patient. A "proximal end" of, for example, the probe includes an end of the probe intended to be near the clinician when the probe is used on the patient. The proximal portion, the proximal end portion, or the proximal length of the probe can include the proximal end of the probe; however, the proximal portion, the proximal end portion, or the proximal length of the probe need not include the proximal end of the probe. That is, unless context suggests otherwise, the proximal portion, the proximal end portion, or the proximal length of the probe is not a terminal portion or terminal length of the probe.

With respect to "distal," a "distal portion" or a "distal end portion" of, for example, a probe disclosed herein includes a portion of the probe intended to be near or in a patient when the probe is used on the patient. Likewise, a "distal length" of, for example, the probe includes a length of the probe intended to be near or in the patient when the probe is used on the patient. A "distal end" of, for example, the probe includes an end of the probe intended to be near or in the patient when the probe is used on the patient. The distal portion, the distal end portion, or the distal length of the probe can include the distal end of the probe; however, the distal portion, the distal end portion, or the distal length of the probe need not include the distal end of the probe. That is, unless context suggests otherwise, the distal portion, the distal end portion, or the distal length of the probe is not a terminal portion or terminal length of the probe.

The term "logic" may be representative of hardware, firmware or software that is configured to perform one or more functions. As hardware, the term logic may refer to or include circuitry having data processing and/or storage functionality. Examples of such circuitry may include, but are not limited or restricted to a hardware processor (e.g., microprocessor, one or more processor cores, a digital signal processor, a programmable gate array, a microcontroller, an application specific integrated circuit "ASIC", etc.), a semiconductor memory, or combinatorial elements.

Additionally, or in the alternative, the term logic may refer to or include software such as one or more processes, one or more instances, Application Programming Interface(s) (API), subroutine(s), function(s), applet(s), servlet(s), routine(s), source code, object code, shared library/dynamic link library (dll), or even one or more instructions. This software may be stored in any type of a suitable non-transitory storage medium, or transitory storage medium (e.g., electrical, optical, acoustical or other form of propagated signals such as carrier waves, infrared signals, or digital signals). Examples of a non-transitory storage medium may include, but are not limited or restricted to a programmable circuit; non-persistent storage such as volatile memory (e.g., any type of random access memory "RAM"); or persistent storage such as non-volatile memory (e.g., read-only memory "ROM", power-backed RAM, flash memory, phase-change memory, etc.), a solid-state drive, hard disk drive, an optical disc drive, or a portable memory device. As firmware, the logic may be stored in persistent storage.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method.

The phrases "connected to" and "coupled with" refer to any form of interaction between two or more entities, including mechanical, electrical, magnetic, electromagnetic, fluid, and optical interaction. Two components may be connected to or coupled with each other even though they are not in direct contact with each other. For example, two components may be coupled with each other through an intermediate component.

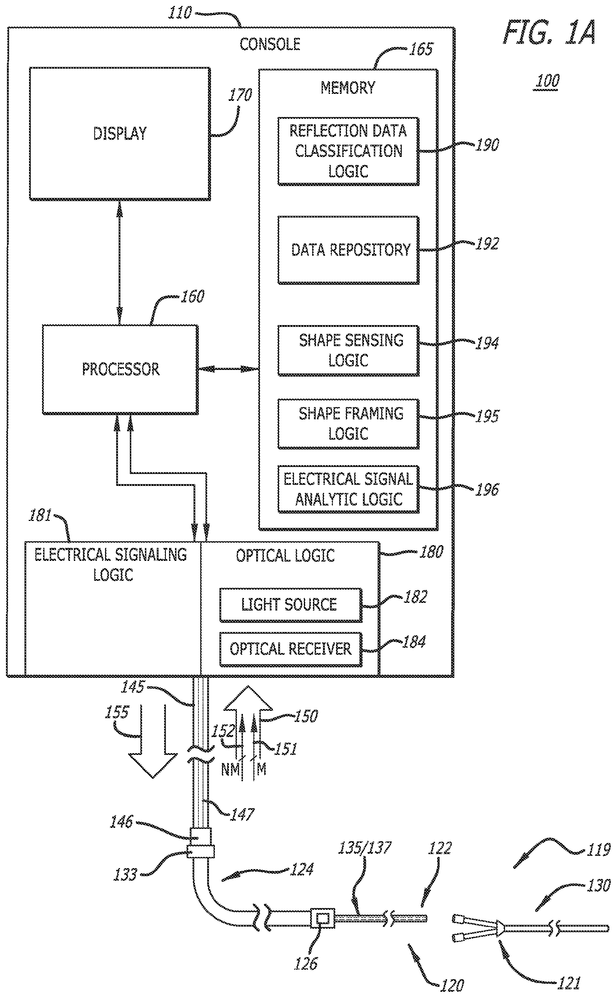

Referring to FIG. 1A, an illustrative embodiment of a medical instrument monitoring system including a medical instrument (sometimes referred to herein as a medical device) with optic shape sensing and fiber optic-based oximetry capabilities is shown in accordance with some embodiments. As shown, the system 100 generally includes a console 110 and a medical device in the form of a stylet assembly 119 communicatively coupled to the console 110. For this embodiment, the stylet assembly 119 includes an elongate probe (e.g., stylet) 120 on its distal end 122 and a console connector 133 on its proximal end 124. The console connector 133 enables the stylet assembly 119 to be operably connected to the console 110 via an interconnect 145 including one or more optical fibers 147 (hereinafter, "optical fiber(s)") and a conductive medium terminated by a single optical/electric connector 146 (or terminated by dual connectors. Herein, the connector 146 is configured to engage (mate) with the console connector 133 to allow for the propagation of light between the console 110 and the stylet assembly 119 as well as the propagation of electrical signals from the stylet 120 to the console 110.

An exemplary implementation of the console 110 includes a processor 160, a memory 165, a display 170 and optical logic 180, although it is appreciated that the console 110 can take one of a variety of forms and may include additional components (e.g., power supplies, ports, interfaces, etc.) that are not directed to aspects of the disclosure. An illustrative example of the console 110 is illustrated in U.S. Patent No. 10,992,078. The processor 160, with access to the memory 165 (e.g., non-volatile memory or non-transitory, computer-readable medium), is included to control functionality of the console 110 during operation. As shown, the display 170 may be a liquid crystal diode (LCD) display integrated into the console 110 and employed as a user interface to display information to the clinician, especially during a catheter placement procedure (e.g., cardiac catheterization). In another embodiment, the display 170 may be separate from the console 110. Although not shown, a user interface is configured to provide user control of the console 110.

In further embodiments, as an alternative to the display 170, the console 110 may be coupled with a virtual reality or augmented reality system (not shown). Such a system may provide an enhanced visualization of the 3D representations shown and described below.

For both of these embodiments, the content depicted by the display 170 may change according to which mode the stylet 120 is configured to operate: optical, TLS, ECG, or another modality. In TLS mode, the content rendered by the display 170 may constitute a two-dimensional (2D) or three-dimensional (3D) representation of the physical state (e.g., length, shape, form, and/or orientation) of the stylet 120 computed from characteristics of reflected light signals 150 returned to the console 110. The reflected light signals 150 constitute light of a specific spectral width of broadband incident light 155 reflected back to the console 110. According to one embodiment of the disclosure, the reflected light signals 150 may pertain to various discrete portions (e.g., specific spectral widths) of broadband incident light 155 transmitted from and sourced by the optical logic 180, as described below

According to one embodiment of the disclosure, an activation control 126, included on the stylet assembly 119, may be used to set the stylet 120 into a desired operating mode and selectively alter operability of the display 170 by the clinician to assist in medical device placement. For example, based on the modality of the stylet 120, the display 170 of the console 110 can be employed for optical modality-based guidance during catheter advancement through the vasculature or TLS modality to determine the physical state (e.g., length, form, shape, orientation, etc.) of the stylet 120. In one embodiment, information from multiple modes, such as optical, TLS or ECG for example, may be displayed concurrently (e.g., at least partially overlapping in time).

Referring still to FIG. 1A, the optical logic 180 is configured to support operability of the stylet assembly 119 and enable the return of information to the console 110, which may be used to determine the physical state associated with the stylet 120 along with monitored electrical signals such as ECG signaling via an electrical signaling logic 181 that supports receipt and processing of the received electrical signals from the stylet 120 (e.g., ports, analog-to-digital conversion logic, etc.). The physical state of the stylet 120 may be based on changes in characteristics of the reflected light signals 150 received at the console 110 from the stylet 120. The characteristics may include shifts in wavelength caused by strain on certain regions of the core fibers integrated within an optical fiber core 135 positioned within or operating as the stylet 120, as shown below. As discussed herein, the optical fiber core 135 may be comprised of core fibers 137₁-137_{M} (M=1 for a single core, and M≥2 for a multi-core), where the core fibers 137₁-137_{M} may collectively be referred to as core fiber(s) 137. Unless otherwise specified or the instant embodiment requires an alternative interpretation, embodiments discussed herein will refer to a multi-core optical fiber 135. From information associated with the reflected light signals 150, the console 110 may determine (through computation or extrapolation of the wavelength shifts) the physical state of the stylet 120, and also that of a catheter 121 configured to receive the stylet 120.

According to one embodiment of the disclosure, as shown in FIG. 1A, the optical logic 180 may include a light source 182 and an optical receiver 184. The light source 182 is configured to transmit the incident light 155 (e.g., broadband) for propagation over the optical fiber(s) 147 included in the interconnect 145, which are optically connected to the multi-core optical fiber core 135 within the stylet 120. In one embodiment, the light source 182 is a tunable swept laser, although other suitable light sources can also be employed in addition to a laser, including semi-coherent light sources, LED light sources, etc.

The optical receiver 184 is configured to: (i) receive returned optical signals, namely reflected light signals 150 received from optical fiber-based reflective gratings (sensors) fabricated within each core fiber of the multi-core optical fiber 135 deployed within the stylet 120, and (ii) translate the reflected light signals 150 into reflection data (from repository 192), namely data in the form of electrical signals representative of the reflected light signals including wavelength shifts caused by strain. The reflected light signals 150 associated with different spectral widths may include reflected light signals 151 provided from sensors positioned in the center core fiber (reference) of the multi-core optical fiber 135 and reflected light signals 152 provided from sensors positioned in the periphery core fibers of the multi-core optical fiber 135, as described below. Herein, the optical receiver 184 may be implemented as a photodetector, such as a positive-intrinsic-negative "PIN" photodiode, avalanche photodiode, or the like.

As shown, both the light source 182 and the optical receiver 184 are operably connected to the processor 160, which governs their operation. Also, the optical receiver 184 is operably coupled to provide the reflection data (from repository 192) to the memory 165 for storage and processing by reflection data classification logic 190. The reflection data classification logic 190 may be configured to: (i) identify which core fibers pertain to which of the received reflection data (from repository 192) and (ii) segregate the reflection data stored with a repository 192 provided from reflected light signals 150 pertaining to similar regions of the stylet 120 or spectral widths into analysis groups. The reflection data for each analysis group is made available to shape sensing logic 194 for analytics.

According to one embodiment of the disclosure, the shape sensing logic 194 is configured to compare wavelength shifts measured by sensors deployed in each periphery core fiber at the same measurement region of the stylet 120 (or same spectral width) to the wavelength shift at a center core fiber of the multi-core optical fiber 135 positioned along central axis and operating as a neutral axis of bending. From these analytics, the shape sensing logic 194 may determine the shape the core fibers have taken in 3D space and may further determine the current physical state of the catheter 121 in 3D space for rendering on the display 170.

According to one embodiment of the disclosure, the shape sensing logic 194 may generate a rendering of the current physical state of the stylet 120 (and potentially the catheter 121), based on heuristics or run-time analytics. For example, the shape sensing logic 194 may be configured in accordance with machine-learning techniques to access a data store (library) with pre-stored data (e.g., images, etc.) pertaining to different regions of the stylet 120 (or catheter 121) in which reflected light from core fibers have previously experienced similar or identical wavelength shifts. From the pre-stored data, the current physical state of the stylet 120 (or catheter 121) may be rendered. Alternatively, as another example, the shape sensing logic 194 may be configured to determine, during run-time, changes in the physical state of each region of the multi-core optical fiber 135 based on at least: (i) resultant wavelength shifts experienced by different core fibers within the optical fiber 135, and (ii) the relationship of these wavelength shifts generated by sensors positioned along different periphery core fibers at the same cross-sectional region of the multi-core optical fiber 135 to the wavelength shift generated by a sensor of the center core fiber at the same cross-sectional region. It is contemplated that other processes and procedures may be performed to utilize the wavelength shifts as measured by sensors along each of the core fibers within the multi-core optical fiber 135 to render appropriate changes in the physical state of the stylet 120 (and/or catheter 121), especially to enable guidance of the stylet 120, when positioned at a distal tip of the catheter 121, within the vasculature of the patient and at a desired destination within the body.

The console 110 may further include electrical signaling logic 181, which is positioned to receive one or more electrical signals from the stylet 120. The stylet 120 is configured to support both optical connectivity as well as electrical connectivity. The electrical signaling logic 181 receives the electrical signals (e.g., ECG signals) from the stylet 120 via the conductive medium. The electrical signals may be processed by electrical signal logic 196, executed by the processor 160, to determine ECG waveforms for display.

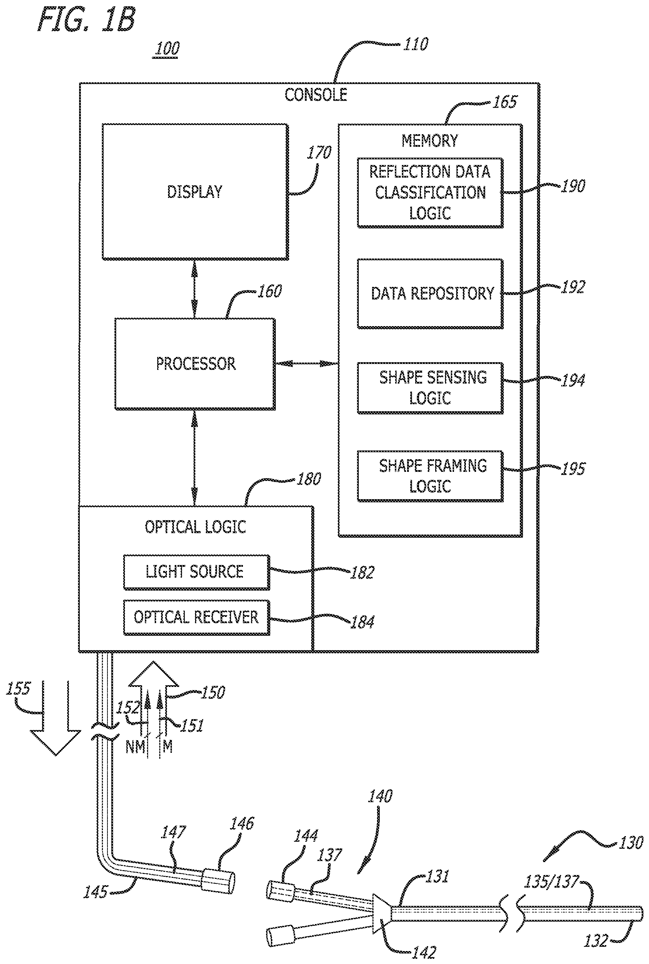

Referring to FIG. 1B, an alternative exemplary embodiment of a medical instrument monitoring system 100 is shown. Herein, the medical instrument monitoring system 100 features a console 110 and a medical instrument 130 communicatively coupled to the console 110. For this embodiment, the medical instrument 130 corresponds to a catheter, which features an integrated tubing with two or more lumen extending between a proximal end 131 and a distal end 132 of the integrated tubing. The integrated tubing (sometimes referred to as "catheter tubing") is in communication with one or more extension legs 140 via a bifurcation hub 142. An optical-based catheter connector 144 may be included on a proximal end of at least one of the extension legs 140 to enable the catheter 130 to operably connect to the console 110 via an interconnect 145 or another suitable component. Herein, the interconnect 145 may include a connector 146 that, when coupled to the optical-based catheter connector 144, establishes optical connectivity between one or more optical fibers 147 (hereinafter, "optical fiber(s)") included as part of the interconnect 145 and core fibers 137 deployed within the catheter 130 and integrated into the tubing. Alternatively, a different combination of connectors, including one or more adapters, may be used to optically connect the optical fiber(s) 147 to the core fibers 137 within the catheter 130. The core fibers 137 deployed within the catheter 130 as illustrated in FIG. 1B include the same characteristics and perform the same functionalities as the core fibers 137 deployed within the stylet 120 of FIG. 1A.

The optical logic 180 is configured to support graphical rendering of the catheter 130, most notably the integrated tubing of the catheter 130, based on characteristics of the reflected light signals 150 received from the catheter 130. The characteristics may include shifts in wavelength caused by strain on certain regions of the core fibers 137 integrated within (or along) a wall of the integrated tubing, which may be used to determine (through computation or extrapolation of the wavelength shifts) the physical state of the catheter 130, notably its integrated tubing or a portion of the integrated tubing such as a tip or distal end.

More specifically, the optical logic 180 includes a light source 182. The light source 182 is configured to transmit the broadband incident light 155 for propagation over the optical fiber(s) 147 included in the interconnect 145, which are optically connected to multiple core fibers 137 within the catheter tubing. Herein, the optical receiver 184 is configured to: (i) receive returned optical signals, namely reflected light signals 150 received from optical fiber-based reflective gratings (sensors) fabricated within each of the core fibers 137 deployed within the catheter 130, and (ii) translate the reflected light signals 150 into reflection data (from repository 192), namely data in the form of electrical signals representative of the reflected light signals including wavelength shifts caused by strain. The reflected light signals 150 associated with different spectral widths include reflected light signals 151 provided from sensors positioned in the center core fiber (reference) of the catheter 130 and reflected light signals 152 provided from sensors positioned in the outer core fibers of the catheter 130, as described below.

As noted above, the shape sensing logic 194 is configured to compare wavelength shifts measured by sensors deployed in each outer core fiber at the same measurement region of the catheter (or same spectral width) to the wavelength shift at the center core fiber positioned along central axis and operating as a neutral axis of bending. From these analytics, the shape sensing logic 194 may determine the shape the core fibers have taken in 3D space and may further determine the current physical state of the catheter 130 in 3D space for rendering on the display 170.

According to one embodiment of the disclosure, the shape sensing logic 194 may generate a rendering of the current physical state of the catheter 130, especially the integrated tubing, based on heuristics or run-time analytics. For example, the shape sensing logic 194 may be configured in accordance with machine-learning techniques to access a data store (library) with pre-stored data (e.g., images, etc.) pertaining to different regions of the catheter 130 in which the core fibers 137 experienced similar or identical wavelength shifts. From the pre-stored data, the current physical state of the catheter 130 may be rendered. Alternatively, as another example, the shape sensing logic 194 may be configured to determine, during run-time, changes in the physical state of each region of the catheter 130, notably the tubing, based on at least (i) resultant wavelength shifts experienced by the core fibers 137 and (ii) the relationship of these wavelength shifts generated by sensors positioned along different outer core fibers at the same cross-sectional region of the catheter 130 to the wavelength shift generated by a sensor of the center core fiber at the same cross-sectional region. It is contemplated that other processes and procedures may be performed to utilize the wavelength shifts as measured by sensors along each of the core fibers 137 to render appropriate changes in the physical state of the catheter 130.

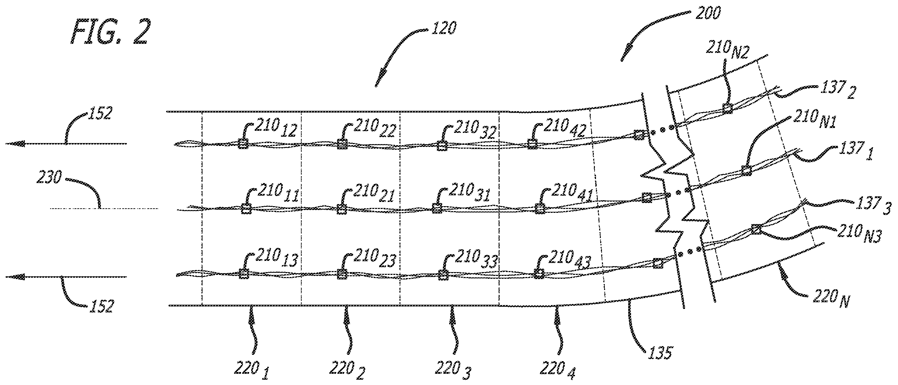

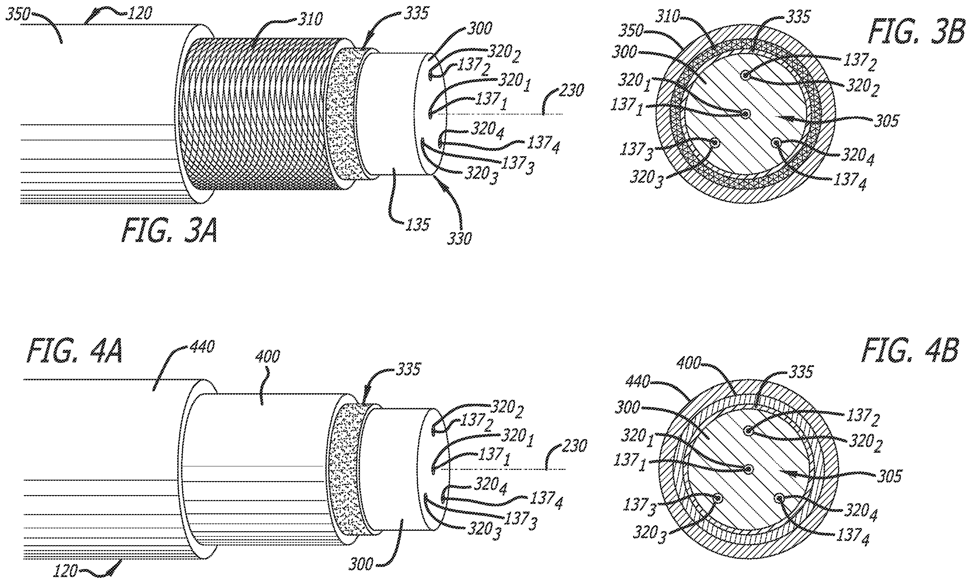

Referring to FIG. 2, an exemplary embodiment of a structure of a section of the multi-core optical fiber included within the stylet 120 of FIG. 1A is shown in accordance with some embodiments. The multi-core optical fiber section 200 of the multi-core optical fiber 135 depicts certain core fibers 137₁-137_{M} (M22, M=4 as shown, see FIG. 3A) along with the spatial relationship between sensors (e.g., reflective gratings) 210₁₁-210_{NM} (N≥2; M≥2) present within the core fibers 137₁-137_{M}, respectively. As noted above, the core fibers 137₁-137_{M} may be collectively referred to as "the core fibers 137."

As shown, the section 200 is subdivided into a plurality of cross-sectional regions 220₁-220_{N}, where each cross-sectional region 220₁-220_{N} corresponds to reflective gratings 210₁₁-210₁₄...210_{N1}-210_{N4}. Some or all of the cross-sectional regions 220₁...220_{N} may be static (e.g., prescribed length) or may be dynamic (e.g., vary in size among the regions 220₁...220_{N}). A first core fiber 137₁ is positioned substantially along a center (neutral) axis 230 while core fiber 137₂ may be oriented within the cladding of the multi-core optical fiber 135, from a cross-sectional, front-facing perspective, to be position on "top" the first core fiber 137₁. In this deployment, the core fibers 137₃ and 137₄ may be positioned "bottom left" and "bottom right" of the first core fiber 137₁. As examples, FIGS. 3A-4B provides illustrations of such.

Referencing the first core fiber 137₁ as an illustrative example, when the stylet 120 is operative, each of the reflective gratings 210₁-210_{N} reflects light for a different spectral width. As shown, each of the gratings 210₁ᵢ-210_{Ni} (1≤i≤M) is associated with a different, specific spectral width, which would be represented by different center frequencies of *f*₁...*f*_{N}, where neighboring spectral widths reflected by neighboring gratings are non-overlapping according to one embodiment of the disclosure.

Herein, positioned in different core fibers 137₂-137₃ but along at the same cross-sectional regions 220-220_{N} of the multi-core optical fiber 135, the gratings 210₁₂-210_{N2} and 210₁₃-210_{N3} are configured to reflect incoming light at same (or substantially similar) center frequency. As a result, the reflected light returns information that allows for a determination of the physical state of the optical fibers 137 (and the stylet 120) based on wavelength shifts measured from the returned, reflected light. In particular, strain (e.g., compression or tension) applied to the multi-core optical fiber 135 (e.g., at least core fibers 137₂-137₃) results in wavelength shifts associated with the returned, reflected light. Based on different locations, the core fibers 137₁-137₄ experience different types and degree of strain based on angular path changes as the stylet 120 advances in the patient.

For example, with respect to the multi-core optical fiber section 200 of FIG. 2, in response to angular (e.g., radial) movement of the stylet 120 is in the left-veering direction, the fourth core fiber 137₄ (see FIG. 3A) of the multi-core optical fiber 135 with the shortest radius during movement (e.g., core fiber closest to a direction of angular change) would exhibit compression (e.g., forces to shorten length). At the same time, the third core fiber 137₃ with the longest radius during movement (e.g., core fiber furthest from the direction of angular change) would exhibit tension (e.g., forces to increase length). As these forces are different and unequal, the reflected light from reflective gratings 210_{N2} and 210_{N3} associated with the core fiber 137₂ and 137₃ will exhibit different changes in wavelength. The differences in wavelength shift of the reflected light signals 150 can be used to extrapolate the physical configuration of the stylet 120 by determining the degrees of wavelength change caused by compression/tension for each of the periphery fibers (e.g., the second core fiber 137₂ and the third core fiber 137₃) in comparison to the wavelength of the reference core fiber (e.g., first core fiber 137₁) located along the neutral axis 230 of the multi-core optical fiber 135. These degrees of wavelength change may be used to extrapolate the physical state of the stylet 120. The reflected light signals 150 are reflected back to the console 110 via individual paths over a particular core fiber 137₁-137_{M}.

Referring to FIG. 3A, a first exemplary embodiment of the stylet of FIG. 1A supporting both an optical and electrical signaling is shown in accordance with some embodiments. Herein, the stylet 120 features a centrally located multi-core optical fiber 135, which includes a cladding 300 and a plurality of core fibers 137₁-137_{M} (M≥2; M=4) residing within a corresponding plurality of lumens 320₁-320_{M}. While the multi-core optical fiber 135 is illustrated within four (4) core fibers 137₁-137₄, a greater number of core fibers 137₁-137_{M} (M>4) may be deployed to provide a more detailed 3D sensing of the physical state (e.g., shape, etc.) of the multi-core optical fiber 135 and the stylet 120 deploying the optical fiber 135.

For this embodiment of the disclosure, the multi-core optical fiber 135 is encapsulated within a concentric braided tubing 310 positioned over a low coefficient of friction layer 335. The braided tubing 310 may feature a "mesh" construction, in which the spacing between the intersecting conductive elements is selected based on the degree of rigidity desired for the stylet 120, as a greater spacing may provide a lesser rigidity, and thereby, a more pliable stylet 120.

According to this embodiment of the disclosure, as shown in FIGS. 3A-3B, the core fibers 137₁-137₄ include (i) a central core fiber 137₁ and (ii) a plurality of periphery core fibers 137₂-137₄, which are maintained within lumens 320₁-320₄ formed in the cladding 300. According to one embodiment of the disclosure, one or more of the lumen 320₁-320₄ may be configured with a diameter sized to be greater than the diameter of the core fibers 137₁-137₄. By avoiding a majority of the surface area of the core fibers 137₁-137₄ from being in direct physical contact with a wall surface of the lumens 320₁-320₄, the wavelength changes to the incident light are caused by angular deviations in the multi-core optical fiber 135 thereby reducing influence of compression and tension forces being applied to the walls of the lumens 320₁-320_{M}, not the core fibers 137₁-137_{M} themselves.

As further shown in FIGS. 3A-3B, the core fibers 137₁-137₄ may include central core fiber 137₁ residing within a first lumen 320₁ formed along the first neutral axis 230 and a plurality of core fibers 137₂-137₄ residing within lumens 320₂-320₄ each formed within different areas of the cladding 300 radiating from the first neutral axis 230. In general, the core fibers 137₂-137₄, exclusive of the central core fiber 137₁, may be positioned at different areas within a cross-sectional area 305 of the cladding 300 to provide sufficient separation to enable 3D sensing of the multi-core optical fiber 135 based on changes in wavelength of incident light propagating through the core fibers 137₂-137₄ and reflected back to the console for analysis.

For example, where the cladding 300 features a circular cross-sectional area 305 as shown in FIG. 3B, the core fibers 137₂-137₄ may be positioned substantially equidistant from each other as measured along a perimeter of the cladding 300, such as at "top" (12 o'clock), "bottom-left" (8 o'clock) and "bottom-right" (4 o'clock) locations as shown. Hence, in general terms, the core fibers 137₂-137₄ may be positioned within different segments of the cross-sectional area 305. Where the cross-sectional area 305 of the cladding 300 has a distal tip 330 and features a polygon cross-sectional shape (e.g., triangular, square, rectangular, pentagon, hexagon, octagon, etc.), the central core fiber 137₁ may be located at or near a center of the polygon shape, while the remaining core fibers 137₂-137_{M} may be located proximate to angles between intersecting sides of the polygon shape.

Referring still to FIGS. 3A-3B, operating as the conductive medium for the stylet 120, the braided tubing 310 provides mechanical integrity to the multi-core optical fiber 135 and operates as a conductive pathway for electrical signals. For example, the braided tubing 310 may be exposed to a distal tip of the stylet 120. The cladding 300 and the braided tubing 310, which is positioned concentrically surrounding a circumference of the cladding 300, are contained within the same insulating layer 350. The insulating layer 350 may be a sheath or conduit made of protective, insulating (e.g., non-conductive) material that encapsulates both for the cladding 300 and the braided tubing 310, as shown.

Referring to FIG. 4A, a second exemplary embodiment of the stylet of FIG. 1B is shown in accordance with some embodiments. Referring now to FIG. 4A, a second exemplary embodiment of the stylet 120 of FIG. 1B supporting both an optical and electrical signaling is shown. Herein, the stylet 120 features the multi-core optical fiber 135 described above and shown in FIG. 3A, which includes the cladding 300 and the first plurality of core fibers 137₁-137_{M} (M≥3; M=4 for embodiment) residing within the corresponding plurality of lumens 320₁-320_{M}. For this embodiment of the disclosure, the multi-core optical fiber 135 includes the central core fiber 137₁ residing within the first lumen 320₁ formed along the first neutral axis 230 and the second plurality of core fibers 137₂-137₄ residing within corresponding lumens 320₂-320₄ positioned in different segments within the cross-sectional area 305 of the cladding 300. Herein, the multi-core optical fiber 135 is encapsulated within a conductive tubing 400. The conductive tubing 400 may feature a "hollow" conductive cylindrical member concentrically encapsulating the multi-core optical fiber 135.

Referring to FIGS. 4A-4B, operating as a conductive medium for the stylet 120 in the transfer of electrical signals (e.g., ECG signals) to the console, the conductive tubing 400 may be exposed up to a tip 410 of the stylet 120. For this embodiment of the disclosure, a conductive epoxy 420 (e.g., metal-based epoxy such as a silver epoxy) may be affixed to the tip 410 and similarly joined with a termination/connection point created at a proximal end 430 of the stylet 120. The cladding 300 and the conductive tubing 400, which is positioned concentrically surrounding a circumference of the cladding 300, are contained within the same insulating layer 440. The insulating layer 440 may be a protective conduit encapsulating both for the cladding 300 and the conductive tubing 400, as shown.

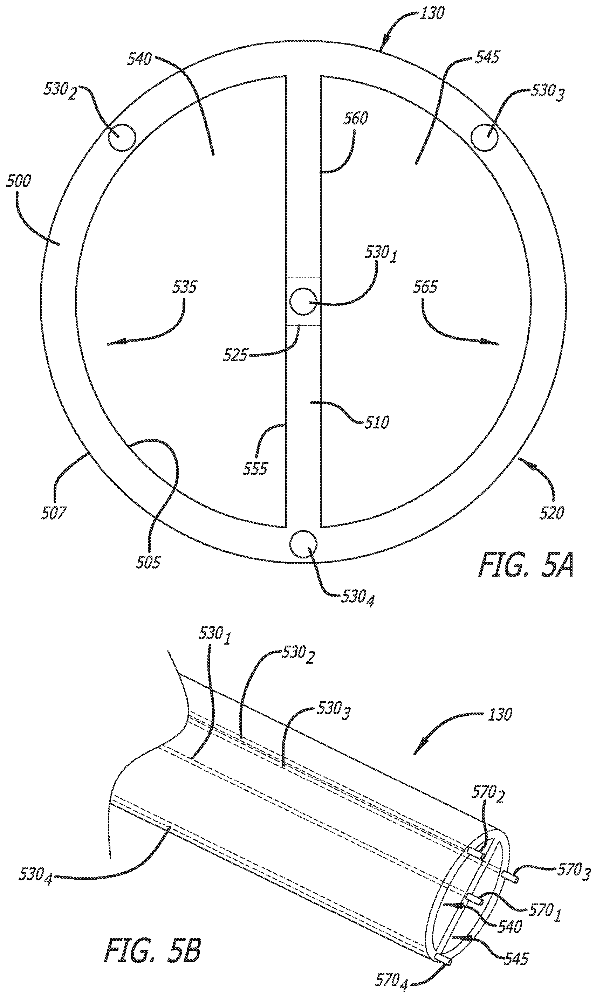

Referring to FIG. 5A, an elevation view of a first illustrative embodiment of a catheter including integrated tubing, a diametrically disposed septum, and micro-lumens formed within the tubing and septum is shown in accordance with some embodiments. Herein, the catheter 130 includes integrated tubing, the diametrically disposed septum 510, and the plurality of micro-lumens 530₁-530₄ which, for this embodiment, are fabricated to reside within the wall 500 of the integrated tubing of the catheter 130 and within the septum 510. In particular, the septum 510 separates a single lumen, formed by the inner surface 505 of the wall 500 of the catheter 130, into multiple lumen, namely two lumens 540 and 545 as shown. Herein, the first lumen 540 is formed between a first arc-shaped portion 535 of the inner surface 505 of the wall 500 forming the catheter 130 and a first outer surface 555 of the septum 510 extending longitudinally within the catheter 130. The second lumen 545 is formed between a second arc-shaped portion 565 of the inner surface 505 of the wall 500 forming the catheter 130 and a second outer surfaces 560 of the septum 510.

According to one embodiment of the disclosure, the two lumens 540 and 545 have approximately the same volume. However, the septum 510 need not separate the tubing into two equal lumens. For example, instead of the septum 510 extending vertically (12 o'clock to 6 o'clock) from a front-facing, cross-sectional perspective of the tubing, the septum 510 could extend horizontally (3 o'clock to 9 o'clock), diagonally (1 o'clock to 7 o'clock; 10 o'clock to 4 o'clock) or angularly (2 o'clock to 10 o'clock). In the later configuration, each of the lumens 540 and 545 of the catheter 130 would have a different volume.

With respect to the plurality of micro-lumens 530₁-530₄, the first micro-lumen 530₁ is fabricated within the septum 510 at or near the cross-sectional center 525 of the integrated tubing. For this embodiment, three micro-lumens 530₂-530₄ are fabricated to reside within the wall 500 of the catheter 130. In particular, a second micro-lumen 530₂ is fabricated within the wall 500 of the catheter 130, namely between the inner surface 505 and outer surface 507 of the first arc-shaped portion 535 of the wall 500. Similarly, the third micro-lumen 530₃ is also fabricated within the wall 500 of the catheter 130, namely between the inner and outer surfaces 505/507 of the second arc-shaped portion 555 of the wall 500. The fourth micro-lumen 530₄ is also fabricated within the inner and outer surfaces 505/507 of the wall 500 that are aligned with the septum 510.

According to one embodiment of the disclosure, as shown in FIG. 5A, the micro-lumens 530₂-530₄ are positioned in accordance with a "top-left" (10 o'clock), "top-right" (2 o'clock) and "bottom" (6 o'clock) layout from a front-facing, cross-sectional perspective. Of course, the micro-lumens 530₂-530₄ may be positioned differently, provided that the micro-lumens 530₂-530₄ are spatially separated along the circumference 520 of the catheter 130 to ensure a more robust collection of reflected light signals from the outer core fibers 570₂-570₄ when installed. For example, two or more of micro-lumens (e.g., micro-lumens 530₂ and 530₄) may be positioned at different quadrants along the circumference 520 of the catheter wall 500.

Referring to FIG. 5B, a perspective view of the first illustrative embodiment of the catheter of FIG. 5A including core fibers installed within the micro-lumens is shown in accordance with some embodiments. According to one embodiment of the disclosure, the second plurality of micro-lumens 530₂-530₄ are sized to retain corresponding outer core fibers 570₂-570₄, where the diameter of each of the second plurality of micro-lumens 530₂-530₄ may be sized just larger than the diameters of the outer core fibers 570₂-570₄. The size differences between a diameter of a single core fiber and a diameter of any of the micro-lumen 530₁-530₄ may range between 0.001 micrometers (µm) and 1000 µm, for example. As a result, the cross-sectional areas of the outer core fibers 570₂-570₄ would be less than the cross-sectional areas of the corresponding micro-lumens 530₂-530₄. A "larger" micro-lumen (e.g., micro-lumen 530₂) may better isolate external strain being applied to the outer core fiber 570₂ from strain directly applied to the catheter 130 itself. Similarly, the first micro-lumen 530₁ may be sized to retain the center core fiber 570₁, where the diameter of the first micro-lumen 530₁ may be sized just larger than the diameter of the center core fiber 570₁.

As an alternative embodiment of the disclosure, one or more of the micro-lumens 530₁-530₄ may be sized with a diameter that exceeds the diameter of the corresponding one or more core fibers 570₁-570₄. However, at least one of the micro-lumens 530₁-530₄ is sized to fixedly retain their corresponding core fiber (e.g., core fiber retained with no spacing between its lateral surface and the interior wall surface of its corresponding micro-lumen). As yet another alternative embodiment of the disclosure, all the micro-lumens 530₁-530₄ are sized with a diameter to fixedly retain the core fibers 570₁-570₄.

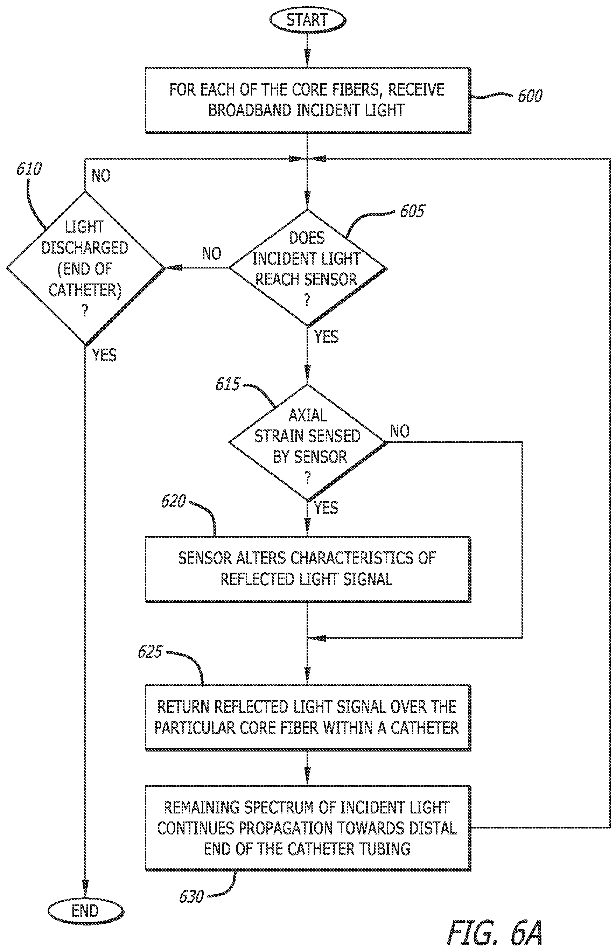

Referring to FIGS. 6A-6B, flowcharts of methods of operations conducted by the medical instrument monitoring system of FIGS. 1A-1B to achieve optic 3D shape sensing are shown in accordance with some embodiments. Herein, the catheter includes at least one septum spanning across a diameter of the tubing wall and continuing longitudinally to subdivide the tubing wall. The medial portion of the septum is fabricated with a first micro-lumen, where the first micro-lumen is coaxial with the central axis of the catheter tubing. The first micro-lumen is configured to retain a center core fiber. Two or more micro-lumen, other than the first micro-lumen, are positioned at different locations circumferentially spaced along the wall of the catheter tubing. For example, two or more of the second plurality of micro-lumens may be positioned at different quadrants along the circumference of the catheter wall.

Furthermore, each core fiber includes a plurality of sensors spatially distributed along its length between at least the proximal and distal ends of the catheter tubing. This array of sensors is distributed to position sensors at different regions of the core fiber to enable distributed measurements of strain throughout the entire length or a selected portion of the catheter tubing. These distributed measurements may be conveyed through reflected light of different spectral widths (e.g., specific wavelength or specific wavelength ranges) that undergoes certain wavelength shifts based on the type and degree of strain.

According to one embodiment of the disclosure, as shown in FIG. 6A, for each core fiber, broadband incident light is supplied to propagate through a particular core fiber (block 600). Unless discharged, upon the incident light reaching a sensor of a distributed array of sensors measuring strain on a particular core fiber, light of a prescribed spectral width associated with the first sensor is to be reflected back to an optical receiver within a console (blocks 605-610). Herein, the sensor alters characteristics of the reflected light signal to identify the type and degree of strain on the particular core fiber as measured by the first sensor (blocks 615-620). According to one embodiment of the disclosure, the alteration in characteristics of the reflected light signal may signify a change (shift) in the wavelength of the reflected light signal from the wavelength of the incident light signal associated with the prescribed spectral width. The sensor returns the reflected light signal over the core fiber and the remaining spectrum of the incident light continues propagation through the core fiber toward a distal end of the catheter tubing (blocks 625-630). The remaining spectrum of the incident light may encounter other sensors of the distributed array of sensors, where each of these sensors would operate as set forth in blocks 605-630 until the last sensor of the distributed array of sensors returns the reflected light signal associated with its assigned spectral width and the remaining spectrum is discharged as illumination.

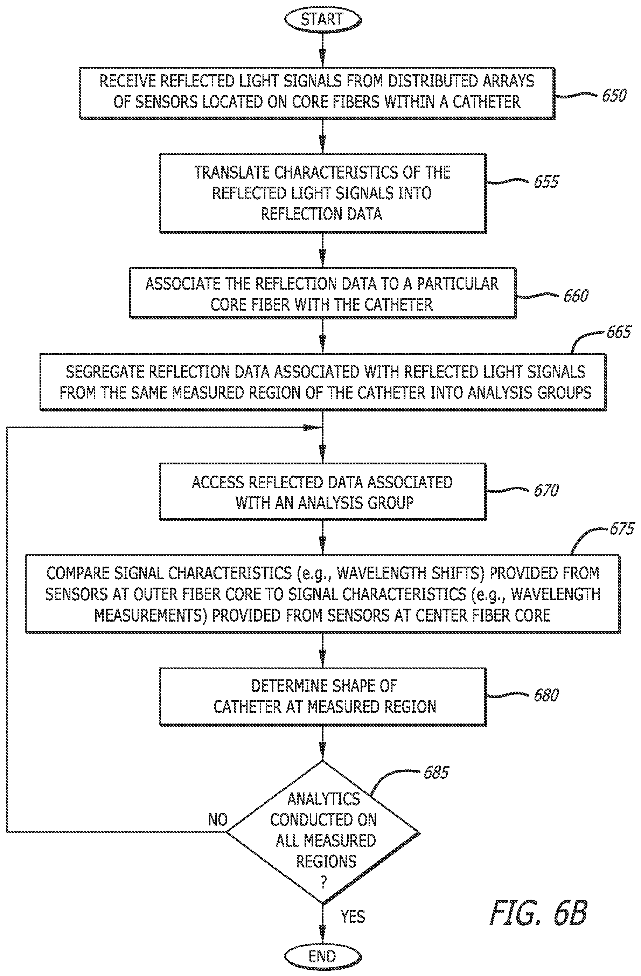

Referring now to FIG. 6B, during operation, multiple reflected light signals are returned to the console from each of the plurality of core fibers residing within the corresponding plurality of micro-lumens formed within a catheter, such as the catheter of FIG. 1B. In particular, the optical receiver receives reflected light signals from the distributed arrays of sensors located on the center core fiber and the outer core fibers and translates the reflected light signals into reflection data, namely electrical signals representative of the reflected light signals including wavelength shifts caused by strain (blocks 650-655). The reflection data classification logic is configured to identify which core fibers pertain to which reflection data and segregate reflection data provided from reflected light signals pertaining to a particular measurement region (or similar spectral width) into analysis groups (block 660-665).

Each analysis group of reflection data is provided to shape sensing logic for analytics (block 670). Herein, the shape sensing logic compares wavelength shifts at each outer core fiber with the wavelength shift at the center core fiber positioned along central axis and operating as a neutral axis of bending (block 675). From this analytics, on all analytic groups (e.g., reflected light signals from sensors in all or most of the core fibers), the shape sensing logic may determine the shape the core fibers have taken in 3D space, from which the shape sensing logic can determine the current physical state of the catheter in three-dimension space (blocks 680-685).

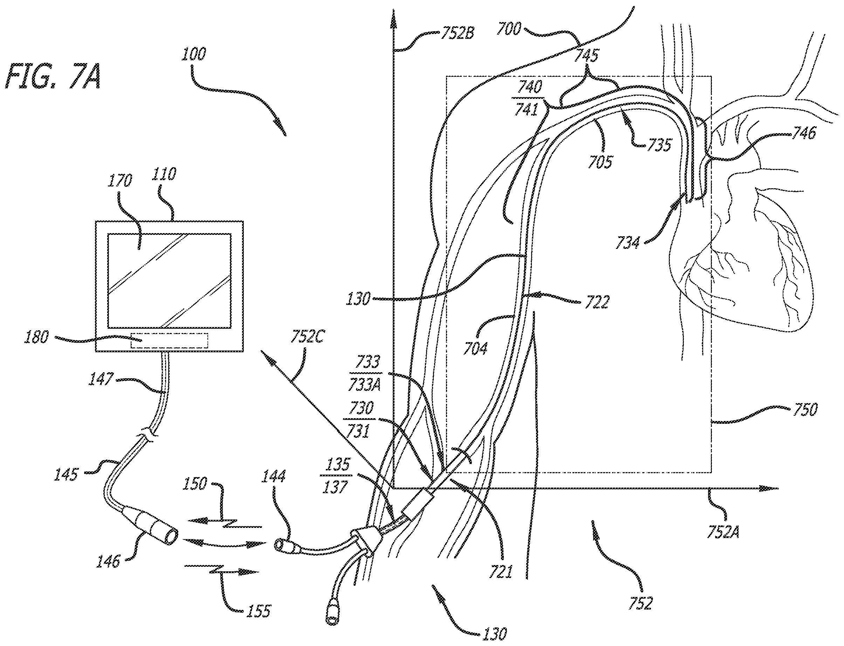

Referring to FIG. 7A, an exemplary embodiment of the medical instrument monitoring system of FIG. 1B during operation and insertion of the catheter into a patient is shown in accordance with some embodiments. For illustrative purposes, FIG. 7A includes the 3D coordinate axis system 752 having a horizontal x-axis 752A pointing to the right, a vertical y-axis 752B pointing up, and a z-axis 752C pointing into the page to illustrate the 3D space surrounding the patient 700. Herein, the catheter 130 generally includes the integrated tubing of the catheter 130. A proximal portion 721 generally remains exterior to the patient 700 and a distal portion 722 generally resides within the patient 700 after placement is complete. The (integrated) catheter tubing of the catheter 130 may be advanced to a desired position within a patient vasculature 704 such that a distal end (or tip) 734 of the catheter tubing of the catheter 130 is proximate the patient's heart, such as in the lower one-third (1/3) portion of the Superior Vena Cava ("SVC"), for example. In some embodiments, various instruments may be disposed at the distal end 734 of the catheter 130 to measure pressure of blood in a certain heart chamber and in the blood vessels, view an interior of blood vessels, or the like. In alternative embodiments, such as those that utilize the stylet assembly of FIG. 1A and the catheter 121, such instruments may be disposed at a distal end of the stylet 120.

During advancement through the patient vasculature 704, the catheter tubing of the catheter 130 receives broadband incident light 155 from the console 110 via optical fiber(s) 147 within the interconnect 145, where the incident light 155 propagates along the core fibers 137 of the multi-core optical fiber 135 within the catheter tubing of the catheter 130. According to one embodiment of the disclosure, the connector 146 of the interconnect 145 terminating the optical fiber(s) 147 may be coupled to the optical-based catheter connector 144, which may be configured to terminate the core fibers 137 deployed within the catheter 130. Such coupling optically connects the core fibers 137 of the catheter 130 with the optical fiber(s) 147 within the interconnect 145. The optical connectivity is needed to propagate the incident light 155 to the core fibers 137 and return the reflected light signals 150 to the optical logic 180 within the console 110 over the interconnect 145. As described below in detail, the physical state of the catheter 130 may be ascertained based on analytics of the wavelength shifts of the reflected light signals 150 where the physical state includes a 3D shape 735 of the optical fiber 135. The 3D shape 735 generally comprises a curved line in 3D space consistent with the 3D shape of the catheter 130.

In some embodiments, the system 100 may include a guide 730 having a straight section 731. The guide 730 may be formed of an introducer having a lumen through which the catheter 130 is inserted. In use a distal portion of the guide 730 may be disposed inside the patient 700 while a proximal portion remains outside the patient 700. In some implementations, the guide 730, or more specifically the straight section 731, may facilitate a calibration of the optical fiber 135. For example, while a section 733 of the optical fiber 135 is disposed within the straight section 731, the shape framing logic 195 may interpret shape data pertaining to the shape 733A of the section 733 as defining a straight line.

The shape framing logic 195 may define a reference plane 750 in accordance with a subshape 740 where the subshape 740 is a portion of the 3D shape 735. The shape framing logic 195 may orient the reference plane 750 in the 3D space as represented by the coordinate axis system 752, and shape framing logic 195 may utilize the reference plane 750 for rendering an image of the 3D shape 735 on the display 170.

The shape framing logic 195 analyzes the subshape 740 to determine whether the subshape 740 is consistent in shape with a predetermined subshape that may be stored in the data repository 192. As used herein, the term "consistent in shape" may be that a first subshape and a second subshape match at least within a tolerance for a substantial percentage of the two subshapes. Detecting a predetermined shape, may include comparing the subshape 740 with a selected one of a plurality of stored predetermined subshapes in the memory (e.g., the data repository 192) to determine if the subshape 740 is effectively consistent with the selected one of the stored predetermined subshapes. With the subshape 740 determined to be consistent with a predetermined subshape in memory, the subshape 740 may then be defined as the predetermined subshape 741 of the 3D shape 735.

The plurality of stored predetermined subshapes may include a subset of stored predetermined subshapes pertaining to anatomical pathways through the body of the patient 700. For example, a stored predetermined subshape may pertain to an anatomical pathway of the patient vasculature 704 including one or more of a basilic vein, a subclavian vein, an innominate vein, or a superior vena cava.

Selecting a stored predetermined subshape from the plurality of predetermined subshapes stored in memory may include receiving input from a clinician where the input defines the anatomical pathway. For example, the input may include an insertion site for the medical instrument.

According to one embodiment of the disclosure, the shape framing logic 195 may generate a rendering of the current position and orientation of the catheter 130 based on heuristics or run-time analytics. For example, the shape framing logic 195 may be configured in accordance with machine-learning (ML) techniques to access the stored predetermined shapes in the data repository 192 pertaining to different anatomical pathways of the catheter 130. For example, a machine-learning model may be trained, using the stored predetermined shapes as training data, to generate scores of predetermined subshapes of the catheter 130 disposed along various anatomical pathways (e.g., anatomical pathways of patients of different sizes), where the score represents a probability that a particular predetermined subshape of the 3D shape corresponds to a stored predetermined subshape in the data repository 192. The ML model may be configured to receive predetermined subshape data and provide a score for the predetermined subshape.

In some embodiments, the shape framing logic 195 may define the reference plane 750 in accordance with two or more points or segments of the predetermined subshape 741. According to one embodiment, the shape framing logic 195 may identify the two segments 745, 746 of the predetermined subshape 741 for defining the reference plane 750. The segments 745, 746 may each effectively define a line segment where the segments 745, 746 are not collinear. In some embodiments, the shape framing logic 195 may utilize a cross-product geometric technique to define a normal line that is perpendicular to both segments 745, 746 and then define the reference plane 750 as perpendicular to the normal line. In other embodiments, the shape framing logic 195 may define the reference plane 750 according to a geometric technique utilizing a line defined by one of the two segments 745, 746 and a point disposed along the other segment. In still other embodiments, the shape framing logic 195 may define the reference plane 750 according to a geometric technique utilizing a line defined by one of the two segments 745, 746 and a direction defined by the other segment. As may be appreciated by one of ordinary skill, other geometric techniques may be utilized to define the plane 750 in accordance with the 3D shape 735.

With further reference to FIG. 7A, the catheter 130 is illustrated in accordance with a front view of the patient 700 where the front side of the patient 700 may be parallel with the x-y plane of the coordinate axis system 752. As such, the 3D shape 735 is similarly illustrated in accordance with a front view of the patient 700. In some instances, the reference plane 750 may be substantially parallel with the front side of the patient 700. Therefore, a front view image of the 3D shape 735 may be substantially consistent with viewing the 3D shape 735 at angle perpendicular to the reference plane 750.

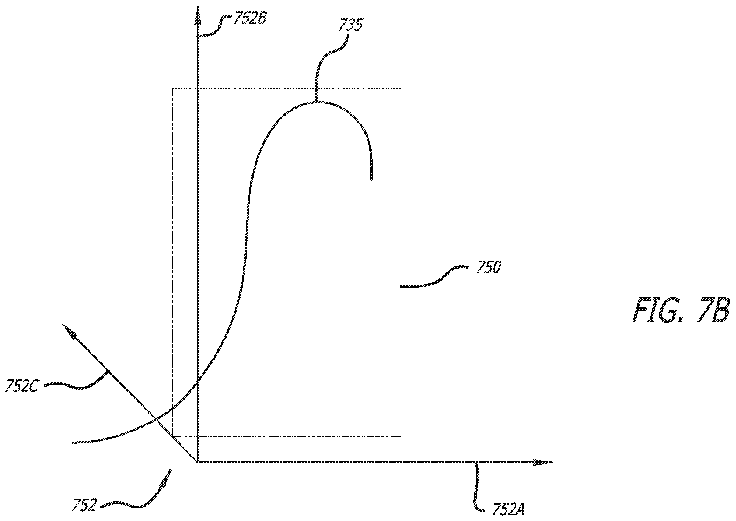

FIG. 7B illustrates the 3D shape 735 in 3D space where the 3D space is again illustrated as the 3D coordinate axis system 752. The shape framing logic 195 may define the reference plane 750 to establish a viewing reference of an image of the 3D shape 735 on the display 170. For illustrative purposes, the reference plane 750 is shown in accordance with the 3D coordinate axis system 752 having a horizontal x-axis 752A pointing to the right, a vertical y-axis 752B pointing up, and a z-axis 752C pointing into the page. In the illustrated embodiment, the reference plane 750 is oriented so that the reference plane 750 is in parallel with the x-y plane. As such, a front view of the 3D shape 735 is defined by a viewing reference along the z-axis 752C.

In some embodiments, the shape framing logic 195 may lock the orientation of the reference plane 750 to the 3D space, i.e., to the illustrated 3D coordinate axis system 752. In other words, once the reference plane 750 is defined, movement/reorientation of the 3D shape 735 in 3D space, as may be caused by movement of the patient 700, may cause a corresponding movement/reorientation of the 3D shape 735 with respect to the reference plane 750.

In further embodiments, the shape framing logic 195 may lock the reference plane 750 to the predetermined subshape 741. As such, the shape framing logic 195 may be configured to render an image of the 3D shape 735 from any viewing reference. In other words, in response to input from the clinician, the shape framing logic 195 may reorient the reference plane 750, to render an image of the 3D shape 735 from a different viewing reference, such as from the top, right side, etc., for example. Viewing the 3D shape 735 from any angle with respect to the reference frame 750 as may be defined by the operator via the input device. In still further embodiments, the shape framing logic 195 may selectively lock the reference plane 750 to the 3D space or the predetermined subshape 741 in accordance with input from the clinician. In still other embodiments, the shape framing logic 195 may continuously define reference plane 750 according to the predetermined subshape 741 during use of the system 100.

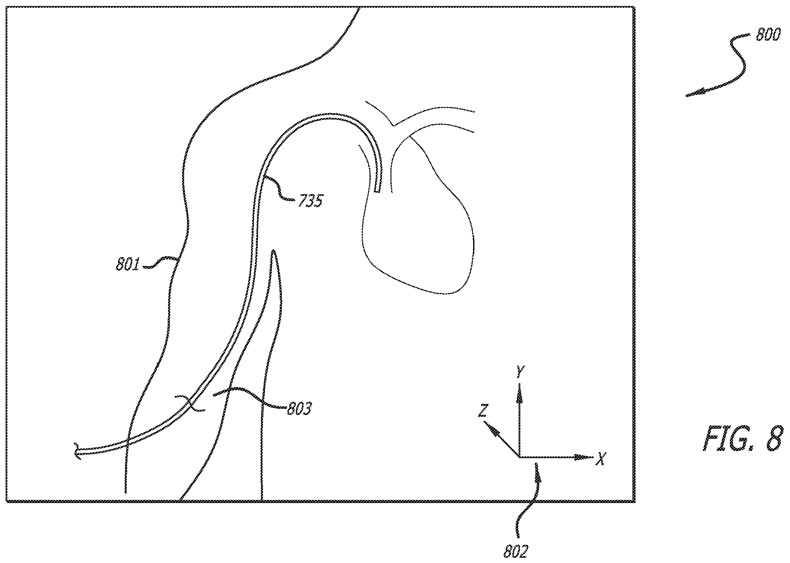

FIG. 8 illustrates an exemplary screen shot 800 showing an image of the 3D shape 735 of FIGS. 7A, 7B rendered according to the reference plane 750. In some embodiments, the screen shot 800 may include a representation 801 of a patient body including the catheter insertion site 803. For example, in the illustrated embodiment, the representation 801 includes an outline of a typical patient body as may be viewed from the front to indicate the orientation of the 3D shape 735. In some embodiments, the representation 801 may include representations of other body parts, such as the heart as illustrated. In still other embodiments, the screen shot 800 may include indicia 801 to indicate the orientation of the 3D shape 735 as defined by the reference frame 751, such as a coordinate axis system, for example. In the screen shot 800, the image of the 3D shape 735 is consistent with the reference plane 750 in parallel with the screen of the display 170 defining a front view of the 3D shape 735. However, images of the 3D shape 735 are not limited to the front view. Although not shown, the shape framing logic 195 may facilitate rendering of images of the 3D shape 735 at any orientation (from any viewing reference) via input from the clinician as discussed above. As such, the shape framing logic 195 may shift the orientation of the representation 801 and/or the indica 802 to provide indication to the clinician as to the rendered orientation of the 3D shape 735.

In some embodiments, the system 100 may be communicatively coupled with an imaging system (e.g., ultrasound, MRI, X-ray, etc., not shown), and the shape framing logic 195 may facilitate rendering the image of the 3D shape 735 along with an image of patient 700. In some instances, the clinician may orient and/or position the image of the 3D shape 735 to position a portion of the 3D shape 735, such as a catheter tip 735, for example, at a specific location relative to an image of the patient 700. As the imaging system may include an image of the medical device directly, such an image may facilitate visual comparison between the 3D shape 735 and the image of the medical device. In some embodiments, the shape framing logic 195 may align a portion the 3D shape 735 with a corresponding portion of the medical device within the image of the patient 700.

In further embodiments, other device location or tracking modalities may be coupled with the system 100 and employed to indicate a position of the catheter 130. Such modalities may include ECG signal monitoring as described above and magnetic field sensing such as described in U.S. Pat. No. 5,099,845 entitled "Medical Instrument Location Means". As such, the system 100 may render images or information on the display 170 pertaining to device location or tracking data in combination with the image of the 3D shape 735.

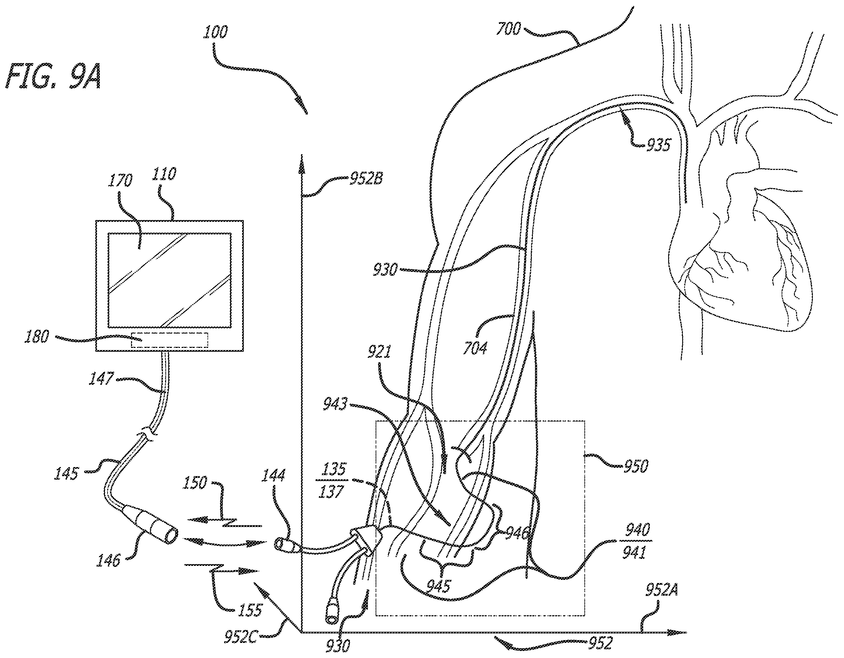

FIG. 9A illustrates second exemplary implementation of the system 100 of FIGS. 1A-1B. The catheter 930 may in some respects resemble the catheter 130 of FIGS. 1A, 1B, 5A, 5B, and 7A. As such, the features and functionalities of the catheter 930 may be the same or similar to the features and functionalities of the catheter 130. The implementation of FIG. 9A differs from the implementation of FIG. 7A in that the predetermined subshape pertains to a portion of the optical fiber disposed exterior to the patient. For illustrative purposes, FIG. 9A includes the 3D coordinate axis system 952 having a horizontal x-axis 952A pointing to the right, a vertical y-axis 952B pointing up, and a z-axis 952C pointing into the page to illustrate the 3D space surrounding the patient 700.

As shown in FIG. 9A, a subshape 940 extends along a portion of the catheter 930 disposed external to the patient 700. The shape framing logic 195 may define a reference plane 950 in accordance with the subshape 940 of the 3D shape 935. Similar to the implementation of FIG. 7A, the shape framing logic 195 analyzes the subshape 940 to determine whether the subshape 940 is consistent in shape with a predetermined subshape that may be stored in the data repository 192. Detecting a predetermined shape, may include comparing the subshape 940 with a selected one of a plurality of stored predetermined subshapes in the memory (e.g., the data repository 192) to determine if the subshape 940 is effectively consistent with the selected one of the stored predetermined subshapes. With the subshape 940 determined to be consistent with a predetermined subshape in memory, the subshape 940 may then be defined as the predetermined subshape 941 of the 3D shape 935.

The plurality of stored predetermined subshapes may include a subset of stored predetermined subshapes pertaining to a proximal portion 921 of the catheter 930 external to the patient 700. Selecting a stored predetermined subshape from the plurality of predetermined stored in memory may include receiving input from a clinician. For example, in some embodiments, the catheter 930 may include a guide for defining a pathway for the catheter 930, where the guide defines the subshape 940. In some embodiments, the clinician may form a bend 943 in the catheter 930 along the proximal portion 921 where the bend defines the subshape 940.

The shape framing logic 195 may utilize the segments 945, 946 to define the reference plane 950 via geometric techniques as described above in relation to defining the reference plane 750 of FIG. 7A above.

In some embodiments, the clinician may position/orient the proximal portion 921 in relation to the patient 700. More specifically, the clinician may orient the subshape 940 to be in parallel with the patient 700 so that the reference plane 950 is in parallel with the patient 700.

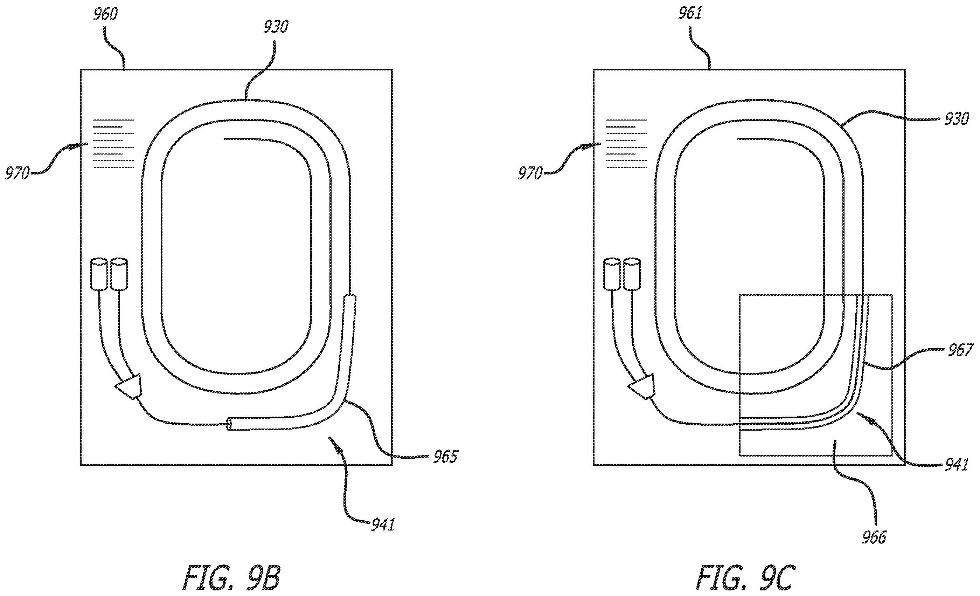

FIG. 9B illustrates a first embodiment of a package 960 containing the catheter 930. The package 960 may be formed of a bag, a pouch, or a hard sided container. The package 960 includes a tubular guide 965 for the catheter 930. The tubular guide 965 is configured to define the predetermined subshape 941. In some embodiments, the catheter 930 is pre-threaded through the tubular guide 965 in a closed state of the package 960 as illustrated. In other embodiments, the catheter 930 may not be pre-threaded through the tubular guide 965, and the clinician may thread the tubular guide 965 onto the catheter 930 after opening the package 960. In some embodiments, the predetermined subshape 941, defined by the tubular guide 965, may be associated with a part number (or other identification information) of the catheter 930, and the shape framing logic 195 may select a stored predetermined shape from memory in accordance with the clinician inputting the part number. In some embodiments, the tubular guide 965 may be integral to the package 960, such as formed in a side wall of the package 960, for example. In some embodiments, the package 960 or a potion thereof may be configured for attachment to the patient so that an orientation of the tubular guide 965 is stable with respect to the patient.

FIG. 9C illustrates a second embodiment of a package 961 containing the catheter 930. The package 961 may be formed of a bag, a pouch, or a hard sided container. The package 961 includes guide plate 966 for the catheter 930. The guide plate 966 includes a groove 967 configured to define the predetermined subshape 941. In some embodiments, the catheter 930 is pre-attached to the guide plate 966 so that catheter 930 is loaded within the groove 967 in a closed state of the package 961 as illustrated. In other embodiments, the catheter 930 may not be pre-loaded within the groove 967, in which case, the clinician may place the catheter 930 within the groove 967 after opening the package 961. In some embodiments, the predetermined subshape 941, defined by the guide plate 966, may be associated with a part number (or other identification information) of the catheter 930, and the shape framing logic 195 may select a stored predetermined shape from memory in accordance with the clinician inputting the part number. In some embodiments, the guide plate 966 may be integral to the package 961, such as formed on a side wall of the package 960, for example. In some embodiments, the guide plate 966 may be separable from the package 961 and may be configured for attachment to the patient so that an orientation of the guide plate 966 is stable with respect to the patient.

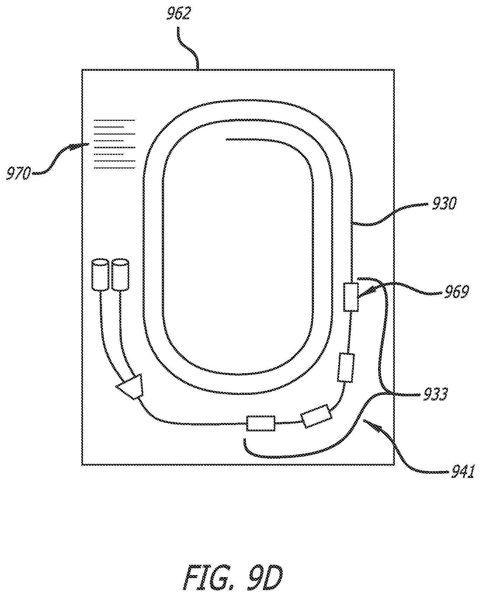

FIG. 9D illustrates a third embodiment of a package 962 containing the catheter 930. The package 962 may be formed of a bag, a pouch, or a hard sided container. In the illustrated embodiment, a portion 933 of the catheter 930 is attached to the package 962 to define the predetermined subshape 941. In some embodiments, the catheter 930 may be continuously attached to a wall of the package 962, or the catheter 930 may be attached at a plurality of the attachment points 969. The attachment mechanism may include an adhesive or structural components (e.g., clips) configured to retain the portion 933 of the catheter 930 at the predetermined subshape 941.

Each of the package embodiments of FIGS. 9B-9D may include package indicia 970. The package indicia 970 may include information relating the predetermined subshape 941. For example, the package indicia 970 may include a part number/model number of the catheter 930 or any other identifying information. The package indicia 970 may include a machine-readable medium such as a bar code, matrix code, RFID, etc. In use, the clinician may input information from the package indicia 970 into the system 100, and the shape framing logic 195 may select a stored predefined shape in memory in accordance with the information from the package indica 970.

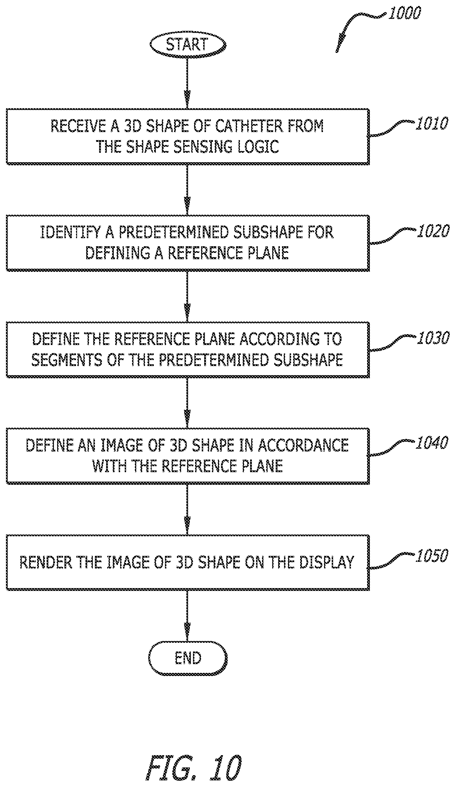

Referring to FIG. 10, a flowchart of a method of operations conducted by the medical instrument monitoring system of FIGS. 1A-1B to render an image of the 3D shape on the display is shown, in accordance with some embodiments. The method 1000 may be performed by the shape framing logic 195. In some embodiments, portions of the method 1000 may be performed by the shape sensing logic 194. The method 1000 generally processes shape data received from the shape sensing logic 194 to define images of the 3D shapes determined from optical fibers extending along the medical device or portions thereof. According to one embodiment of the disclosure, as shown in FIG. 10, the shape framing logic 195 receives 3D shape data pertaining to the 3D shape of the catheter from the shape sensing logic 194 (block 1010).

The shape framing logic 195 may identify a predetermined subshape disposed along the 3D shape of the medical devise (i.e., the optical fiber extending along the medical device) suitable for defining a reference plane to be used in rendering an image of the 3D shape on the display (block 1020). The 3D shape may generally include one or more subshapes (portions of the 3D shape) that may be suitable for use as the predetermined subshape and the shape framing logic 195 identifies a predetermined subshape from the one or more subshapes. Identifying the predetermined subshape may include comparing the predetermined subshape with a stored predetermined subshape in memory. In an instance where a subshape of the 3D shape matches (i.e., is sufficiently consistent with) a stored predetermined subshape, the shape framing logic 195 may define the subshape as the predetermined subshape for the 3D shape.

The memory may include a plurality of stored predetermined subshapes. The stored predetermined subshapes may be defined in accordance with different pathways for the medical device when the medical device is in use. For example, the medical may be advanced along an anatomical pathway of the patient, such as a predefined vasculature of the patient. As such, a stored predetermined subshape may be consistent with a subshape of the medical device when the medical device is disposed within the predefined vasculature. By way of further example, the medical device may be disposed along a pathway of a guide external to the patient. As such, a stored predetermined subshape may be consistent with a subshape of the medical device when the medical device is disposed within the pathway of the guide.

In some embodiments, the shape framing logic 195 may analyze the shape data of the 3D shape to determine a subshape consistent with at least one of the stored predetermined subshapes and then select one of the stored predetermined subshapes for comparison. In some embodiments, the shape framing logic 195 may receive input from a clinician to assist in determining a subshape consistent with at least one of the stored predetermined subshapes. The input may include information pertaining to a predefined anatomical pathway or a pathway of a guide.

Once a predetermined subshape for the 3D shape is defined, the shape framing logic 195 may then define the reference plane in accordance with the predetermined subshape (block 1030). Defining the reference plane may include identifying shape segments (i.e., portions) of the predetermined subshape. The shape framing logic 195 may define the reference plane utilizing geometric techniques utilizing two or more shape segments, such as lines or points extending along the predetermined subshape. According to one embodiment, the shape framing logic 195 may identify two shape segments which are substantially linear and which not collinear with each other. The shape framing logic 195 may utilize a cross-product geometric technique to define a normal line that is perpendicular to both shape segments and then define the reference plane perpendicular to the normal line. As may be appreciated by one of ordinary skill, other geometric techniques may be utilized to define the plane from the portions of the predetermined subshape.

The shape framing logic 195 may then define an image of the 3D shape in accordance with the reference plane (block 1040). In other words, the shape framing logic 195 may define an image of the 3D shape that may viewed on the display from one or more viewpoints with respect to the reference plane, i.e., from the front, top, right side, etc. In some embodiments, the shape framing logic 195 may define an image of the 3D shape viewable from any direction with respect the reference plane.

The shape framing logic 195 may then render the image of the 3D shape on the display (block 1050). In some embodiments, the shape framing logic 195 may lock the orientation of the reference plane in the 3D space so that movement/reorientation of the 3D shape, as may be caused by movement of the patient 700, may cause a corresponding movement/reorientation of the 3D shape within the image.

In other embodiments, the shape framing logic 195 may lock the reference plane to the predetermined subshape so that the viewing reference of the 3D shape remains constant in the event of patient movement. In still other embodiments, the shape framing logic 195 may continuously define reference plane according to the predetermined subshape during use of the system. In some embodiments, the shape framing logic 195 may selectively lock the reference plane in 3D space or the predetermined subshape 741 in response to input from the clinician.

## Claims

1. A medical device system comprising:
a medical device (119) comprising an optical fiber (135) having one or more of core fibers (137), each of the one or more core fibers (137) including a plurality of sensors (210) distributed along a longitudinal length of a corresponding core fiber (137), each sensor (210) of the plurality of sensors (210) configured to:
(i) reflect a light signal of a different spectral width based on received incident light, and
(ii) change a characteristic of the reflected light signal based on strain experienced by the optical fiber (135); and
a console (110) including:
a display (170);
one or more processors (160);
optical logic (180) including a light source (182) and an optical receiver (184); and
a non-transitory computer-readable medium (165) having stored thereon logic, when executed by the one or more processors (160), causes operations including:
providing an incident light (155) signal to the optical fiber (135);
receiving reflected light signals (150) of different spectral widths of the incident light (155) by one or more of the plurality of sensors (210);
processing the reflected light signals (150) associated with the one or more of core fibers (137) to determine a three-dimensional (3D) shape (735) of the optical fiber (135);
detecting a predetermined subshape of the 3D shape (735); and
defining a reference plane (750) in accordance with the predetermined subshape, wherein the reference plane (750) is configured to establish a viewing reference of an image of the 3D shape (735) on the display (170); and
orienting the reference plane (750) in a 3D space as represented by a coordinate axis system (752); and
utilizing the reference plane (750) for rendering an image of the 3D shape (735) on the display (170),
wherein the light source (182) is configured to transmit the incident light (155) and the optical receiver (184) is configured to (i) receive the reflected light signals (150), and (ii) translate the reflected light signals (150) into reflection data.

2. The system according to claim 1, wherein detecting the predetermined subshape includes:
comparing a subshape (740) of the 3D shape (735) with a stored predetermined subshape in memory; and
as a result of the comparison, identifying the subshape (740) as the predetermined subshape of the 3D shape (735).

3. The system according to claim 1, wherein:
the memory includes a plurality of stored predetermined subshapes, and
the operations further include
selecting the stored predetermined subshape from the plurality of stored predetermined subshapes in memory.

4. The system according to any of the preceding claims, wherein the predetermined subshape is defined by a predetermined pathway of the medical device (119).

5. The system according to claim 4, wherein:
the medical device (119) is configured for insertion within a patient body, and
the predetermined pathway includes a predetermined anatomical pathway of the medical device (119).

6. The system according to claim 5, wherein the operations further include:
receiving input from a clinician defining the anatomical pathway for the medical device (119); and
selecting the stored predetermined subshape according to the input from the clinician.

7. The system according to claim 6, wherein the input from the clinician includes a location of an insertion site for the medical device (119).

8. The system according to any of claims 5-7, wherein the predetermined anatomical pathway extends along one or more of a basilic vein, a subclavian vein, an innominate vein, an internal jugular vein, an external jugular vein, or a superior vena cava of the patient.

9. The system according to claim 4, wherein the predetermined pathway is external to the patient.

10. The system according to claim 9, wherein:
the predetermined pathway is defined by a subshape guide of the medical device (119), and
in use, the medical device (119) is disposed within a pathway of the subshape guide.

11. The system according to either claim 7 or claim 8, wherein the medical device (119) is configured to be inserted within the pathway of the subshape guide by the clinician, in use.

12. The system according to any of claims 9-11, wherein the subshape guide is included within a package of the medical device (119), preferably wherein the subshape guide is formed integral to the package.

13. The system according to any of claims 10-12, wherein in use, the subshape guide is attached to the patient to maintain an orientation of the predetermined subshape with respect to the patient.

14. The system according to any of the preceding claims, wherein defining the reference plane (750) includes:
identifying a pair of shape segments of the predetermined subshape, and
defining a plane in parallel with both shape segments, and/or the system further comprises:
a device guide (730) including a lumen extending along a straight section (731) of the device guide (730), wherein:
in use, the medical device (119) is inserted within the lumen, and
the operations further include calibrating the optical fiber (135) in accordance with the straight section (731).

15. The system according to any of the preceding claims, wherein the medical device (119) is one of an introducer wire, a guidewire, a stylet, a stylet within a needle, a needle with the optical fiber (135) inlayed into a cannula of the needle or a catheter with the optical fiber (135) inlayed into one or more walls of the catheter, and/or wherein the operations further include rendering an image of the 3D shape (735) on a display (170) of the system in accordance with the reference plane (750).

## Patentansprüche

1. Medizinisches Vorrichtungssystem, umfassend:
eine medizinische Vorrichtung (119), umfassend eine optische Faser (135), die eine oder mehrere Kernfasern (137) aufweist, wobei jede der einen oder mehreren Kernfasern (137) eine Vielzahl von Sensoren (210) einschließt, die entlang einer Längslänge einer entsprechenden Kernfaser (137) verteilt sind, wobei jeder Sensor (210) der Vielzahl von Sensoren (210) ausgebildet ist zum:
(i) Reflektieren eines Lichtsignals einer unterschiedlichen Spektralbreite basierend auf empfangenem einfallendem Licht, und
(ii) Ändern einer Eigenschaft des reflektierten Lichtsignals basierend auf einer von der optischen Faser (135) erfahrenen Dehnung; und
eine Konsole (110), einschließend:
eine Anzeige (170);
ein oder mehrere Prozessoren (160);
eine optische Logik (180), einschließend eine Lichtquelle (182) und einen optischen Empfänger (184); und
ein nicht transitorisches computerlesbares Medium (165), aufweisend eine darauf gespeicherte Logik, die, wenn ausgeführt durch den einen oder die mehreren Prozessoren (160), Operationen veranlasst, einschließend:
Bereitstellen eines Signals aus einfallendem Licht (155) an die optische Faser (135);
Empfangen reflektierter Lichtsignale (150) unterschiedlicher Spektralbreiten des einfallenden Lichts (155) durch einen oder mehrere der Vielzahl von Sensoren (210);
Verarbeiten der reflektierten Lichtsignale (150), die mit der einen oder den mehreren Kernfasern (137) assoziiert sind, um eine dreidimensionale (3D) Gestalt (735) der optischen Faser (135) zu bestimmen;
Detektieren einer vorbestimmten Teilgestalt der 3D-Gestalt (735); und
Definieren einer Referenzebene (750) in Übereinstimmung mit der vorbestimmten Teilgestalt, wobei die Referenzebene (750) ausgebildet ist, um eine Ansichtsreferenz eines Bildes der 3D-Gestalt (735) auf der Anzeige (170) zu etablieren; und
Orientieren der Referenzebene (750) in einem 3D-Raum, wie dargestellt durch ein Koordinatenachsensystem (752); und
Nutzen der Referenzebene (750) zum Rendern eines Bildes der 3D-Gestalt (735) auf der Anzeige (170),
wobei die Lichtquelle (182) ausgebildet ist, das einfallende Licht (155) zu übertragen, und der optische Empfänger (184) ausgebildet ist, um (i) die reflektierten Lichtsignale (150) zu empfangen und (ii) die reflektierten Lichtsignale (150) in Reflexionsdaten umzusetzen.

2. System nach Anspruch 1, wobei Detektieren der vorbestimmten Teilgestalt Folgendes einschließt:
Vergleichen einer Teilgestalt (740) der 3D-Gestalt (735) mit einer gespeicherten, vorbestimmten Teilgestalt im Speicher; und
als ein Ergebnis des Vergleichs, Identifizieren der Teilgestalt (740) als die vorbestimmte Teilgestalt der 3D-Gestalt (735).

3. System nach Anspruch 1, wobei:
der Speicher eine Vielzahl von gespeicherten, vorbestimmten Teilgestalten einschließt, und
die Operationen weiter Folgendes einschließen
Auswählen der gespeicherten, vorbestimmten Teilgestalt aus der Vielzahl von gespeicherten vorbestimmten Teilgestalten im Speicher.

4. System nach einem der vorstehenden Ansprüche, wobei die vorbestimmte Teilgestalt durch einen vorbestimmten Pfadweg der medizinischen Vorrichtung (119) definiert ist.

5. System nach Anspruch 4, wobei:
die medizinische Vorrichtung (119) zum Einführen in einen Patientenkörper ausgebildet ist, und
der vorbestimmte Pfadweg einen vorbestimmten anatomischen Pfadweg der medizinischen Vorrichtung (119) einschließt.

6. System nach Anspruch 5, wobei die Operationen weiter Folgendes einschließen:
Empfangen einer Eingabe von einem Kliniker, die den anatomischen Pfadweg für die medizinische Vorrichtung (119) definiert; und
Auswählen der gespeicherten, vorbestimmten Teilgestalt nach der Eingabe von dem Kliniker.

7. System nach Anspruch 6, wobei die Eingabe von dem Kliniker einen Ort einer Einführstelle für die medizinische Vorrichtung (119) einschließt.

8. System nach einem der Ansprüche 5-7, wobei sich der vorbestimmte anatomische Pfadweg entlang einer oder mehrerer von einer Vena Basilica, einer Vena Subclavia, einer Vena Brachiocephalica, einer Vena Jugularis Interna, einer Vena Jugularis Externa oder einer Vena Cava Superior des Patienten erstreckt.

9. System nach Anspruch 4, wobei der vorbestimmte Pfadweg außerhalb des Patienten ist.

10. System nach Anspruch 9, wobei:
der vorbestimmte Pfadweg durch eine Teilgestaltführung der medizinischen Vorrichtung (119) definiert ist, und
bei Verwendung die medizinische Vorrichtung (119) innerhalb eines Pfadwegs der Teilgestaltführung angeordnet ist.

11. System nach entweder Anspruch 7 oder Anspruch 8, wobei die medizinische Vorrichtung (119) ausgebildet ist, um bei Verwendung durch den Kliniker in den Pfadweg der Teilgestaltführung eingeführt zu werden.

12. System nach einem der Ansprüche 9-11, wobei die Teilgestaltführung innerhalb einer Verpackung der medizinischen Vorrichtung (119) eingeschlossen ist, bevorzugt wobei die Teilgestaltführung integral mit der Verpackung gebildet ist.

13. System nach einem der Ansprüche 10-12, wobei bei Verwendung die Teilgestaltführung an dem Patienten befestigt ist, um eine Orientierung der vorbestimmten Teilgestalt in Bezug auf den Patienten aufrechtzuerhalten.

14. System nach einem der vorstehenden Ansprüche, wobei Definieren der Referenzebene (750) Folgendes einschließt:
Identifizieren eines Paares von Gestaltsegmenten der vorbestimmten Teilgestalt, und
Definieren einer Ebene parallel zu beiden Gestaltsegmenten, und/oder wobei das System weiter Folgendes umfasst:
eine Vorrichtungsführung (730), einschließend ein Lumen, das sich entlang einer geraden Sektion (731) der Vorrichtungsführung (730) erstreckt, wobei:
bei Verwendung die medizinische Vorrichtung (119) in das Lumen eingeführt wird, und
die Operationen weiter Kalibrieren der optischen Faser (135) in Übereinstimmung mit der geraden Sektion (731) einschließen.

15. System nach einem der vorstehenden Ansprüche, wobei die medizinische Vorrichtung (119) eines aus einem Einführungsdraht, einem Führungsdraht, einem Stilett, einem Stilett innerhalb einer Nadel, einer Nadel mit der optischen Faser (135), die in eine Kanüle der Nadel eingelegt ist, oder einem Katheter mit der optischen Faser (135), die in eine oder mehrere Wände des Katheters eingelegt ist, ist und/oder wobei die Operationen weiter Rendern eines Bildes der 3D-Gestalt (735) auf einer Anzeige (170) des Systems in Übereinstimmung mit der Referenzebene (750) einschließen.

## Revendications

1. Système de dispositif médical comprenant :
un dispositif médical (119) comprenant une fibre optique (135) présentant une ou plusieurs fibres à cœur (137), chacune des une ou plusieurs fibres à cœur (137) incluant une pluralité de capteurs (210) répartis le long d'une longueur longitudinale d'une fibre à cœur (137) correspondante, chaque capteur (210) de la pluralité de capteurs (210) étant configuré pour :
(i) réfléchir un signal lumineux d'une largeur spectrale différente sur la base de la lumière incidente reçue, et
(ii) modifier une caractéristique du signal lumineux réfléchi sur la base d'une déformation subie par la fibre optique (135) ; et
une console (110) incluant :
un écran (170) ;
un ou plusieurs processeurs (160) ;
une logique optique (180) incluant une source lumineuse (182) et un récepteur optique (184) ; et
un support non transitoire lisible par ordinateur (165) présentant une logique stockée sur celui-ci, laquelle, lorsqu'elle est exécutée par les un ou plusieurs processeurs (160), provoque des opérations incluant :
la fourniture d'un signal de lumière incidente (155) à la fibre optique (135) ;
la réception de signaux lumineux réfléchis (150) de largeurs spectrales différentes de la lumière incidente (155) par un ou plusieurs de la pluralité de capteurs (210) ;
le traitement des signaux lumineux réfléchis (150) associés aux une ou plusieurs fibres à cœur (137) pour déterminer une forme tridimensionnelle (3D) (735) de la fibre optique (135) ;
la détection d'une sous-forme prédéterminée de la forme 3D (735) ; et
la définition d'un plan de référence (750) conformément à la sous-forme prédéterminée, dans lequel le plan de référence (750) est configuré pour établir une référence de visualisation d'une image de la forme 3D (735) sur l'écran (170) ; et
l'orientation du plan de référence (750) dans un espace 3D tel que représenté par un système d'axes de coordonnées (752) ; et
l'utilisation du plan de référence (750) pour restituer une image de la forme 3D (735) sur l'écran (170),
dans lequel la source lumineuse (182) est configurée pour transmettre la lumière incidente (155) et le récepteur optique (184) est configuré pour (i) recevoir les signaux lumineux réfléchis (150), et (ii) traduire les signaux lumineux réfléchis (150) en données de réflexion.

2. Système selon la revendication 1, dans lequel la détection de la sous-forme prédéterminée inclut :
la comparaison d'une sous-forme (740) de la forme 3D (735) avec une sous-forme prédéterminée stockée dans la mémoire ; et
en conséquence de la comparaison, l'identification de la sous-forme (740) comme étant la sous-forme prédéterminée de la forme 3D (735).

3. Système selon la revendication 1, dans lequel :
la mémoire inclut une pluralité de sous-formes prédéterminées stockées, et
les opérations incluent en outre
la sélection de la sous-forme prédéterminée stockée parmi la pluralité de sous-formes prédéterminées stockées dans la mémoire.

4. Système selon l'une quelconque des revendications précédentes, dans lequel la sous-forme prédéterminée est définie par un trajet prédéterminé du dispositif médical (119).

5. Système selon la revendication 4, dans lequel :
le dispositif médical (119) est configuré pour une insertion à l'intérieur d'un corps de patient, et
le trajet prédéterminé inclut un trajet anatomique prédéterminé du dispositif médical (119).

6. Système selon la revendication 5, dans lequel les opérations incluent en outre :
la réception d'une entrée d'un clinicien définissant le trajet anatomique pour le dispositif médical (119) ; et
la sélection de la sous-forme prédéterminée stockée selon l'entrée du clinicien.

7. Système selon la revendication 6, dans lequel l'entrée du clinicien inclut un emplacement d'un site d'insertion pour le dispositif médical (119).

8. Système selon l'une quelconque des revendications 5 à 7, dans lequel le trajet anatomique prédéterminé s'étend le long d'une ou de plusieurs des veines suivantes du patient : une veine basilique, une veine sous-clavière, une veine innominée, une veine jugulaire interne, une veine jugulaire externe ou une veine cave supérieure.

9. Système selon la revendication 4, dans lequel le trajet prédéterminé est externe au patient.

10. Système selon la revendication 9, dans lequel :
le trajet prédéterminé est défini par un guide de sous-forme du dispositif médical (119), et
en utilisation, le dispositif médical (119) est disposé à l'intérieur d'un trajet du guide de sous-forme.

11. Système selon soit la revendication 7 soit la revendication 8, dans lequel le dispositif médical (119) est configuré pour être inséré dans le trajet du guide de sous-forme par le clinicien, en utilisation.

12. Système selon l'une quelconque des revendications 9 à 11, dans lequel le guide de sous-forme est inclus dans un emballage du dispositif médical (119), de préférence dans lequel le guide de sous-forme est formé d'un seul tenant avec l'emballage.

13. Système selon l'une quelconque des revendications 10 à 12, dans lequel, en utilisation, le guide de sous-forme est fixé au patient pour maintenir une orientation de la sous-forme prédéterminée par rapport au patient.

14. Système selon l'une quelconque des revendications précédentes, dans lequel la définition du plan de référence (750) inclut :
l'identification d'une paire de segments de forme de la sous-forme prédéterminée, et
la définition d'un plan parallèle aux deux segments de forme, et/ou le système comprend en outre :
un guide de dispositif (730) incluant une lumière s'étendant le long d'une section rectiligne (731) du guide de dispositif (730), dans lequel :
en utilisation, le dispositif médical (119) est inséré à l'intérieur de la lumière, et
les opérations incluent en outre l'étalonnage de la fibre optique (135) conformément à la section rectiligne (731).

15. Système selon l'une quelconque des revendications précédentes, dans lequel le dispositif médical (119) est un élément parmi un fil introducteur, un fil-guide, un stylet, un stylet à l'intérieur d'une aiguille, une aiguille avec la fibre optique (135) intégrée dans une canule de l'aiguille ou un cathéter avec la fibre optique (135) intégrée dans une ou plusieurs parois du cathéter, et/ou dans lequel les opérations incluent en outre la restitution d'une image de la forme 3D (735) sur un écran (170) du système conformément au plan de référence (750).
